# FASCICULE DE BREVET EUROPEEN

(11) **EP 0 804 435 B1**
(45) Date de publication et mention de la délivrance du brevet: **10.03.2004**
(21) Numéro de dépôt: 95927003.4
(22) Date de dépôt: 01.08.1995
(51) Int. Cl.: C07D 405/06, A61K 31/415

(54) **NOUVEAUX DERIVES DE L'IMIDAZOLE, LEUR PROCEDE DE PREPARATION, LES NOUVEAUX INTERMEDIAIRES OBTENUS, LEUR APPLICATION A TITRE DE MEDICAMENTS ET LES COMPOSITIONS PHARMACEUTIQUES LES RENFERMANT**
IMIDAZOLDERIVATE, IHRER HERSTELLUNG, ZWISCHENPRODUKTE, UND IHRE VERWENDUNG ALS ARZNEIMITTEL
NOVEL IMIDAZOLE DERIVATIVES, METHOD FOR PREPARING SAME, NOVEL INTERMEDIATES THUS OBTAINED, MEDICINAL APPLICATIONS OF SAME AND PHARMACEUTICAL COMPOSITIONS CONTAINING SAME

(30) Priorité: 02.08.1994 FR 9409565
(43) Date de publication de la demande: 05.11.1997
(73) Titulaire: Aventis Pharma S.A., 92160 Antony (FR)
(72) Inventeur: HECKMANN, Bertrand, F-94230 Cachan (FR); VEVERT, Jean-Paul, F-93500 Pantin (FR); ZHANG, Jidong, F-75013 Paris (FR)
(86) Numéro de dépôt international: PCT/FR1995/001033
(87) Numéro de publication internationale: WO 1996/004275

(56) Documents cités:
- WO-A-94/02474
- FR-A- 2 416 012
- CHEMICAL ABSTRACTS, vol. 103, no. 15, 14 Octobre 1985, Columbus, Ohio, US; abstract no. 116118u, G. STEFANCICH ET AL 'Studies on new psychotropic agents. I. Synthesis and pharmacological activity of derivatives of 5H-imidazo(2,1-c)(1,4)benzodiazepine' page 68 ; & FARMACO, EDIZIONE SCIENTIFICA, vol.40, no.6, 1985, PAVIA IT pages 429 - 441
- JOURNAL OF MEDICINAL CHEMISTRY., vol.18, no.8, 1975, WASHINGTON US pages 833 - 836 K. H. BAGGALEY ET AL 'Hypolipidemic imidazoles'

## Description

La présente invention concerne de nouveaux dérivés de l'imidazole, leur procédé de préparation, les nouveaux intermédiaires obtenus, leur application à titre de médicaments et les compositions pharmaceutiques les renfermant.

Le document WO 94/02474 décrit des dérivés antagonistes de récepteur de l'endothéline.

La présente invention a pour objet les produits de formule (I) : 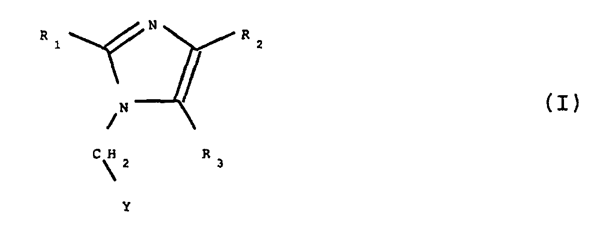 dans laquelle :
R₁ représente l'atome d'hydrogène, le radical alcoxy, 1,3-dioxolanne, dioxanne, phényle, benzoyle et phénylthio dans lesquels le radical phényle est éventuellement substitué par un radical hydroxyle ou alcoxy renfermant au plus 4 atomes de carbone,
R₂ et R₃ identiques ou différents sont choisis parmi les atomes d'halogène ; le radical carboxy libre, salifié ou estérifié par un radical alkyle renfermant au plus 4 atomes de carbone ; le radical formyle ; et les radicaux tétrazolyle, phényle, phénylthio, phénylsulfonyle, phénylsulfinyle, alkyle renfermant au plus 4 atomes de carbone, alkylthio, alkylsulfonyle et alkylsulfinyle, tous ces radicaux étant éventuellement substitués par un ou plusieurs radicaux choisis parmi les atomes d'halogène les radicaux hydroxyle, alcoxy renfermant au plus 4 atomes de carbone, carboxy libre salifié ou estérifié et phényle lui-même éventuellement substitué par un radical hydroxyle ou alcoxy renfermant au plus 4 atomes de carbone,
et Y représente le radical 1,3-benzodioxolyl dont la partie phényle est substituée par un ou plusieurs atomes d'halogène, étant entendu que R₁ ne représente pas un radical dioxolanne
ou dioxanne lorsque R₃ représente un radical carboxy libre ou estérifié ou lorsque R₂ et R₃ représentent un atome d'halogène lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle,
- le terme radical alkynyle linéaire ou ramifié désigne de préférence un radical éthynyle, propargyle, butynyle ou pentynyle.
   Parmi les radicaux alkyles interrompus par un ou plusieurs hétéroatomes, on peut citer par exemple les radicaux méthoxyméthyle, méthoxyéthoxyméthyle, propylthiopropyle, propyloxypropyle, propylthioéthyle, méthylthiométhyle,
- le terme atome d'halogène désigne de préférence l'atome de chlore, mais peut aussi représenter un atome de fluor, de brome ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
   le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle,
- le terme radical acyloxy désigne par exemple un radical dans lequel le radical acyle a les valeurs indiquées ci-dessus et désigne de préférence un radical formyloxy, acétyloxy, propionyloxy, butyryloxy ou benzoyloxy,
- le terme radical cycloalkyle désigne de préférence les radicaux cyclopropyle, cyclobutyle et tout particulièrement les radicaux cyclopentyle et cyclohexyle,
- le terme radical haloalkyle désigne de préférence les radicaux dans lesquels le radical alkyle est tel que défini ci-dessus et est substitué par un ou plusieurs atomes d'halogène tel que défini ci-dessus comme par exemple dans bromoéthyle, trifluorométhyle, trifluoroéthyle ou encore pentafluoroéthyle,
- le terme radical haloalcoxy désigne de préférence les radicaux dans lesquels le radical alcoxy est tel que défini ci-dessus et est substitué par un ou plusieurs atomes d'halogène tel que défini ci-dessus comme par exemple dans bromoéthoxy, trifluorométhoxy, trifluoroéthoxy ou encore pentafluoroéthoxy,
- le terme radical dioxol peut désigner soit un radical carboxycyclique renfermant jusqu'à 6 atomes de carbone et 2 atomes d'oxygène, par exemple le radical 1,3-dioxolan-2-yle ou encore le radical dioxanne, soit un cycle carbocyclique renfermant 2 atomes d'oxygène accolé à un substituant arylique, par exemple le radical 1,3-benzodioxolyle,
- le terme radical alkylthio désigne de préférence les radicaux dans lesquels le radical alkyle est tel que défini ci-dessus comme par exemple dans méthylthio, éthylthio, propylthio, isopropylthio, butylthio, sec-butylthio, tert-butylthio, isopentylthio ou isohexylthio, éventuellement substitué comme par exemple dans hydroxyméthylthio ou aminoéthylthio,
- le terme radical haloalkylthio désigne de préférence les radicaux dans lesquels le radical alkyle est tel que défini ci-dessus et est substitué par un ou plusieurs atomes d'halogène tel que défini ci-dessus comme par exemple dans bromoéthylthio, trifluorométhylthio, trifluoroéthylthio ou encore pentafluoroéthylthio,
- le terme radical arylalkylthio ou alkylthio subtitué par aryle représente par exemple le radical benzylthio ou phénéthylthio.

Dans tous les radicaux que peuvent représenter ou porter R₁, R₂, R₃ et R₄, tels que définis ci-dessus, les atomes de soufre peuvent ne pas être oxydés comme dans les radicaux alkylthio, arylthio, cycloalkylthio tel que par exemple cyclohexylthio ou au contraire être oxydés pour donner les radicaux alkylsulfinyle, cycloalkylsulfinyle, arylsulfinyle, alkylsulfonyle, cycloalkylsulfonyle ou arylsulfonyle :
- les termes radical alkylsulfinyle et alkylsulfonyle désignent les radicaux alkylthio dans lesquels le radical alkyle linéaire ou ramifié peut représenter, par exemple, les valeurs indiquées ci-dessus pour le radical alkyle et dans lesquels le radical thio est oxydé en radical sulfinyle ou sulfonyle. On peut citer par exemple les radicaux méthylsulfinyle, éthylsulfinyle, méthylsulfonyle ou éthylsulfonyle,
- le terme radical arylsulfinyle et arylsulfonyle désigne les radicaux arylthio, dans lesquels le radical aryle peut représenter, par exemple, les valeurs indiquées ci-dessus pour le ràdical aryle et dans lesquels le radical thio est oxydé en radical sulfinyle ou sulfonyle tel que par exemple dans les radicaux phényl-sulfinyle ou -sulfonyle, pyridyl-sulfinyle ou
- sulfonyle, pyrimidyl-sulfinyle ou -sulfonyle, imidazolylsulfinyle ou -sulfonyle ou N-méthylimidazolyl-sulfinyle ou
- sulfonyle.

Les radicaux carbamoyle et amino que peuvent représenter ou porter l'un ou plusieurs des éventuels substituants des radicaux définis dans les produits de formule (I) et dans ce qui suit, désignent des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone, tous ces radicaux étant éventuellement substitués ainsi qu'il est indiqué ci-dessus et ci-après.

Les radicaux carbocycliques ou hétérocycliques que peuvent représenter R₆, R₇, R₈ et R₉ peuvent prendre les valeurs définies ci-dessus pour ces radicaux et en particulier phényle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle.

Lorsque R₆ et R₇ d'une part, R₈ et R₉ d'autre part ou R₁₄ et R₁₅ encore d'autre part, tels que définis ci-dessus, forment ensemble avec l'atome d'azote auquel ils sont liés un hétérocycle, il s'agit, par exemple, d'un cycle pyrrolyle, imidazolyle, indolyle, indolinyle, purinyle, pyrrolidinyle, pipéridino, morpholino, pipérazinyle, imidazolidinyle , pyrazolidinyle, thiomorpholinyle, azépine ; ces radicaux peuvent être éventuellement substitués un ou plusieurs radicaux tels que définis précédemment et en particulier par un ou plusieurs radicaux choisis parmi les atomes de chlore et de fluor,les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, fluorométhylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle, arylalkyle et arylalkényle, comme par exemple dans chlorophényle ou trifluorophényle.

L'hétérocycle que peuvent former R₆ et R₇ d'une part, R₈ et R₉ d'autre part, respectivement avec l'atome d'azote auquel ils sont liés représente de préférence un hétérocycle saturé.

De même, dans les produits de formule (I), les radicaux carbamoyle ou amino sont tels que les radicaux portés par l'atome d'azote sont identiques ou différents, peuvent représenter des chaînes aliphatiques ou cyclisées ou peuvent former avec l'atome d'azote auquel ils sont liés un hétérocycle, ainsi qu'il a été défini ci-dessus pour R₆, R₇, R₈, R₉.

Le radical amino éventuellement substitué désigne ainsi le radical amino éventuellement substitué par un ou deux radicaux alkyle choisis parmi les radicaux alkyle tels que définis ci-dessus comme par exemple pour monoalkylamino dans méthylamino ou éthylamino ou isopropylamino ou par exemple pour dialkylamino dans diméthylamino, diéthylamino ou encore méthyléthylamino, ces radicaux alkyles étant éventuellement substitués ainsi qu'il est indiqué ci-dessus, comme par exemple les radicaux méthoxyméthyle, méthoxyéthyle, éthoxyéthyle.

A titre d'exemple et de façon non exhaustive, lé terme radical carbamoyle éventuellement substitué désigne les radicaux carbamoyle éventuellement substitué sur l'atome d'azote par un ou deux radicaux alkyle éventuellement substitués ainsi qu'il est défini ci-dessus, pour former notamment un groupe N-monoalkyl carbamoyle tel que N-méthylcarbamoyle, N-éthylcarbamoyle ou un groupe N,N-dialkyl carbamoyle, tel que N,N-diméthylcarbamoyle, N,N-diéthylcarbamoyle ; un groupe N-(hydroxyalkyl carbamoyle, tel que N-(hydroxyméthyl) carbamoyle, N-(hydroxyéthyl) carbamoyle, phénylcarbamoyle ; pyridylcarbamoyle ; benzylcarbamoyle ; N-méthyl N-phénylcarbamoyle ; pyridylméthylcarbamoyle. Par ailleurs, parmi les radicaux alkyles substitués, on peut citer également les radicaux alkyles substitués par un radical carbamoyle tel que défini ci-dessus, pour former un groupe carbamoylalkyle tel que carbamoylméthyle ou carbamoyléthyle.

Le radical amino peut être un radical alcoxycarbonylamino, ce radical étant alors de préférence le radical tert-butyloxycarbonylamino ou le radical benzyloxycarbonylamino.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la tri-éthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Lorsque R₂ et R₃ représentent tous deux un groupement soufré, R₂ et R₃ étant identiques ou différents, dans les produits préférés de l'invention, ces groupements soufrés n'ont pas nécessairement le même degré d'oxydation.

L'invention a tout particulièrement pour objet les produits de formule (I) telle que définie ci-dessus, dans laquelle :
R₁ représente l'atome d'hydrogène, le radical 1,3-dioxolanne, phényle, benzoyle et phénylthio dans lesquels le radical phényle est éventuellement substitué par un radical hydroxyle ou alcoxy renfermant au plus 4 atomes de carbone,
R₂ représente un atome d'halogène, un radical alkylthio dans lequel le radical alkyl linéaire ou ramifié, renfermant au plus 4 atomes de carbone, est éventuellement substitué par un radical phényle lui-même éventuellement substitué par un radical alcoxy et le radical phénylthio dans lequel le radical phényle est éventuellement substitué par un radical alcoxy,
R₃ représente un radical formyle ou carboxy libre, estérifié, salifié ou amidifié, et
Y représente un radical phényle substitué par un atome d'halogène et par deux radicaux formant ensemble un radical dioxol,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Parmi les produits objet de l'invention, peuvent être cités tout particulièrement les produits de formule (I) répondant aux formules suivantes :
- 1-((6-chloro-1,3-benzodioxol-5-yl) méthyl)-4-(((4-méthoxyphényl) méthyl) thio)-2-(phénylthio)-1H-imidazole-5-carboxaldéhyde
- acide 4-bromo-1-((6-chloro-1,3-benzodioxol-5-yl) méthyl)-2-phényl-lH-imidazole-5-carboxylique
- acide 1-((6-chloro-1,3-benzodioxol-5-yl) méthyl)-4-(((4-méthoxyphényl) méthyl) thio)-2-(phénylthio)-1H-imidazole-5-carboxylique
- acide 1-((6-chloro-1,3-benzodioxol-5-yl) méthyl)-4-(4-méthoxyphényl) thio)-2-(phénylthio)-1H-imidazole-5-carboxylique
- acide 1-((6-chloro-1,3-benzodioxol-5-yl) méthyl)-2,4-bis(4-(méthoxyphényl) thio)-1H-imidazole-5-carboxylique
- acide 1-((6-chloro 1,3-benzodioxol-5-yl) méthyl) 4-((3,4-diméthoxyphényl) thio) 2-(1,3-dioxolan-2-yl) 1H-imidazol 5-carboxylique,
   ainsi que leurs sels d'addition avec les acides minéraux ou organiques ou avec les bases minérales ou organiques.

L'invention a également pour objet un procédé de préparation des produits de formule (I), telle que définie ci-dessus, caractérisé en ce que :
soit l'on soumet un composé de formule (II) : 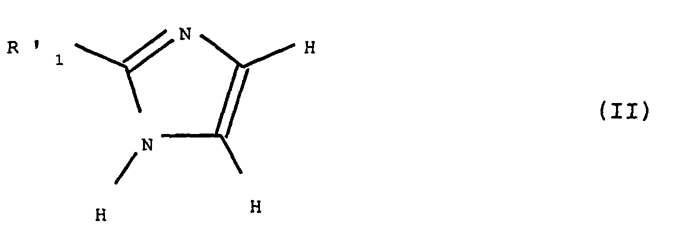
dans laquelle R'₁ a la signification indiquée ci-dessus pour R₁, dans laquelle les éventuelles fonctions réactives sont éventuellement protégées par des groupements protecteurs, à une réaction avec un composé de formule (III) : 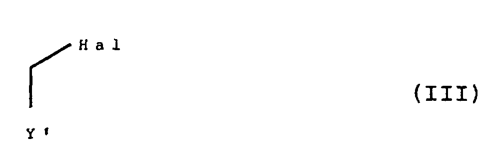 dans laquelle Hal représente un atome d'halogène, et
Y' a la signification indiquée ci-dessus pour Y, dans laquelle les éventuelles fonctions réactives sont éventuellement protégées par des groupements protecteurs, pour obtenir le produit de formule (IV₁) : 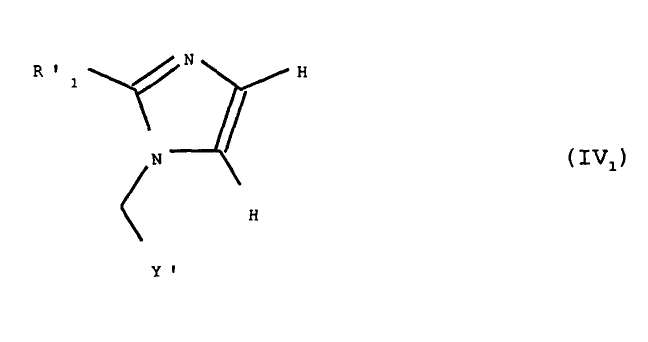 dans laquelle R'₁ et Y' ont les significations indiquées ci-dessus, produit de formule (IV₁) que l'on peut soumettre à une réaction d'halogénation, pour obtenir le produit de formule (IV₂) : 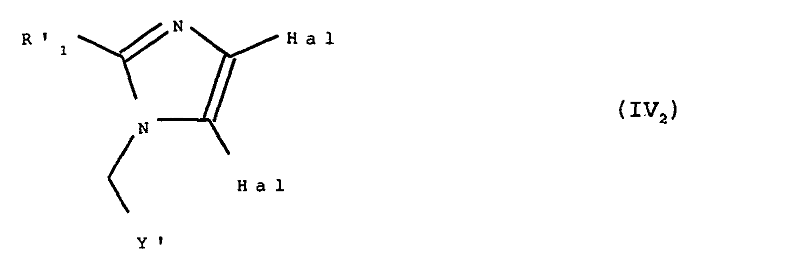 dans laquelle R'₁, Hal et Y' ont les significations indiquées ci-dessus,
soit l'on soumet un composé de formule (VIII) : 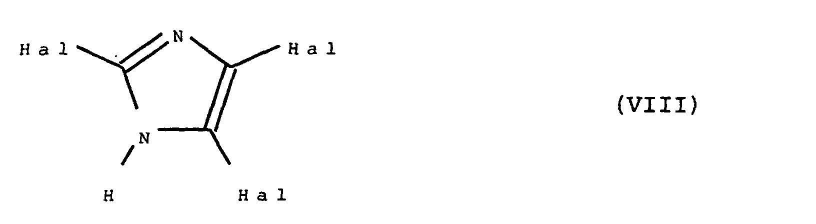 dans laquelle Hal a la signification indiquée ci-dessus,
soit à une réaction avec le composé de formule (III) telle que définie ci-dessus, soit à l'action d'un groupement protecteur P, pour obtenir un produit de formule (IX) : 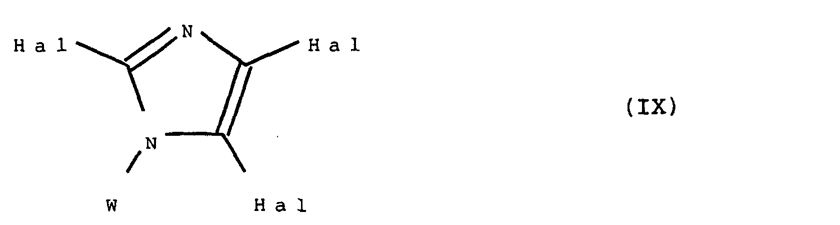 dans laquelle Hal a la signification indiquée ci-dessus et W représente soit -CH₂-Y' avec Y' tel que défini ci-dessus, soit P qui représente un groupement protecteur de l'atome d'azote, produit de formule (IX) que l'on soumet à une réaction d'échange halogène-métal puis à une réaction avec un électrophile de formule (X), (X'), (XI), (XII) ou (XII') :

L₁-CHO (X)

L₁-CO-Cl (X')

(-S-L₁)₂ (XI)

L₁SO₂-S-L'₁ (XII)

L₁SO₂-Cl (XII')

dans lesquelles L₁ et L'₁ identiques ou différents, représentent un radical alkyle, alkényle ou aryle, tels que définis ci-dessus, pour obtenir un produit de formule (XIII) : 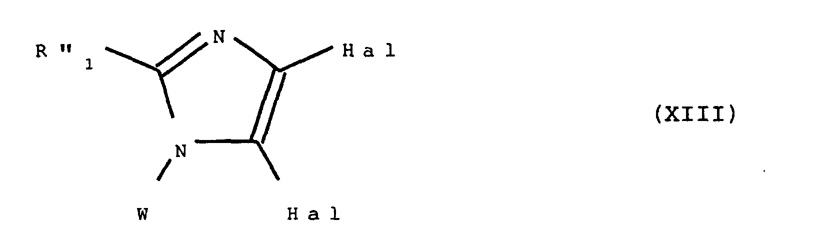 dans laquelle Hal et W ont les significations indiquées ci-dessus, R"₁ représente un radical aryl- ou alkyl-carbonyle, aryl- ou alkyl-hydroxyméthyl ou arylthio dans lesquels les radicaux aryle et alkyle ont les significations indiquées ci-dessus et dans lesquels les éventuelles fonctions réactives sont éventuellement protégées par des groupements protecteurs,
produits de formules (IV₂) et (XIII) que l'on peut soumettre à une réaction d'échange halogène-métal sur l'un des atomes d'halogène puis à une réaction avec CO₂ ou DMF ou un composé électrophile de formule (Vₐ), (V_{b}), (VIₐ), (VI_{b}) ou (VI_{c}) :

(-S-Z₁)₂ (Vₐ)

ou

MeSO₂SZ₁ (V_{b})

ou 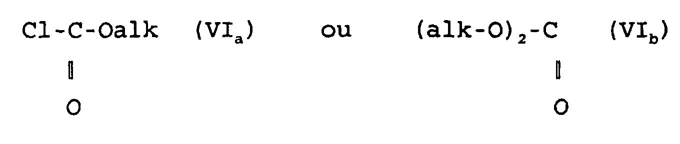 ou 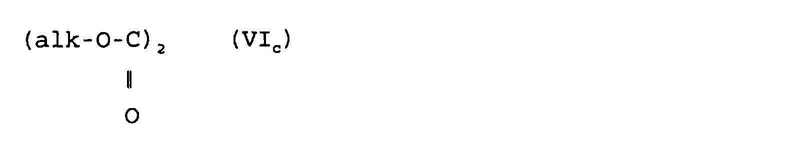 dans lesquelles Z₁ représente un radical alkyle, alkényle ou aryle, éventuellement substitués, dans lesquelles les éventuelles fonctions réactives sont éventuellement protégées par des groupements protecteurs, et alk représente un radical alkyle renfermant au plus 4 atomes de carbone,
pour obtenir respectivement le composé de formule (I₁) : 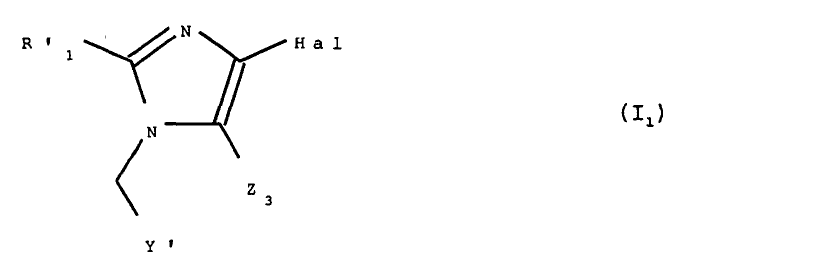 ou le composé de formule (XIV) : 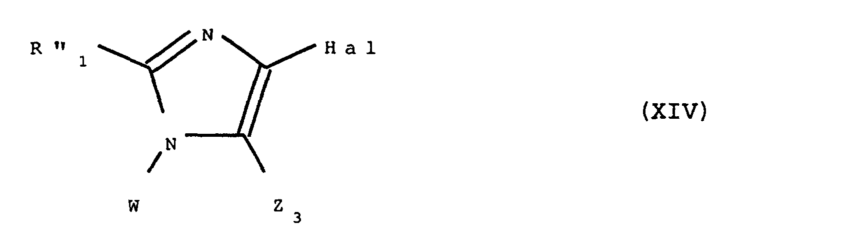 dans lesquelles R'₁, R"₁, Hal, Y' et W ont les significations indiquées ci-dessus et Z₃ représente le radical carboxy, formyle, S-Z₁ tel que défini ci-dessus, ou le radical K-O-alk dans lequel K représente le radical 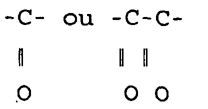 et alk a la signification indiquée ci-dessus, composés de formule (I₁) ou (XIV) que lorsque Z₃ représente un radical formyle ou carboxy estérifié, l'on peut soumettre à une réaction avec un composé de formule (VII) :

Z₂-S-M (VII)

dans laquelle S représente un atome de soufre, M représente un métal tel que sodium, potassium ou cuivre et Z₂ représente un radical alkyle, alkényle ou aryle, éventuellement substitué, dans lesquelles les éventuelles fonctions réactives sont éventuellement protégées par des groupements protecteurs pour obtenir respectivement le composé de formule (I₂) : 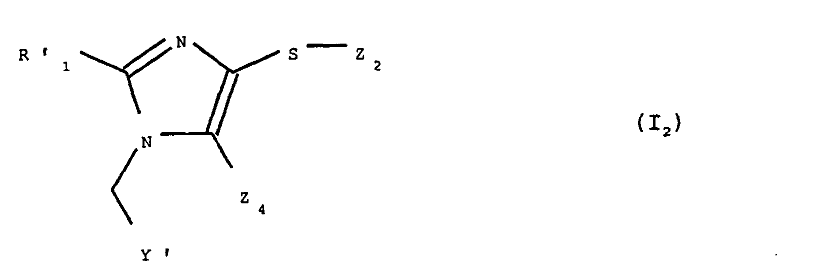 ou le composé de formule (XV) : 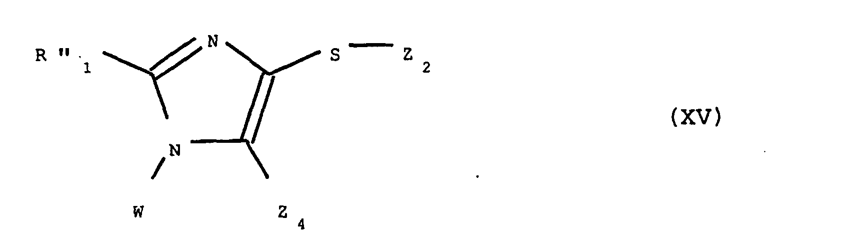 dans lesquelles R'₁, R"₁, Y', Z₂ et W ont les significations indiquées ci-dessus, et Z₄ représente le radical formyle ou carboxy estérifié, produits de formules (I₂) et (XV) que lorsque Z₄ représente le radical formyle, l'on peut soumettre à une réaction d'oxydation ou de réduction pour obtenir respectivement un produit de formule (I₃) : 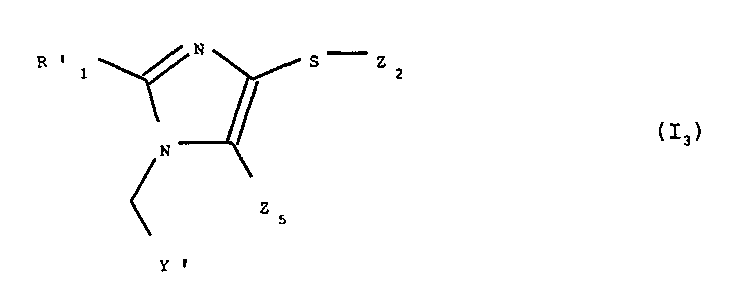 ou un produit de formule (I₄) : 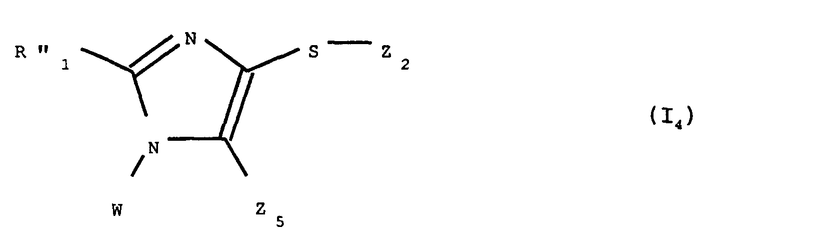 dans lesquelles R'₁, R"₁, Z₂, Y' et W ont les significations indiquées ci-dessus, et Z₅ représente le radical CH₂OH ou le radical carboxy libre ou estérifié par un radical alkyle linéaire ou ramifié renfermant au plus 6 atomes de carbone, produits de formule (XV) ou (I₄) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (I₅) : 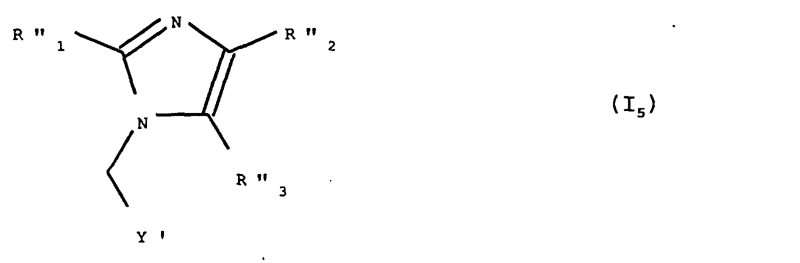 dans laquelle R"₁ et Y' ont les significations indiquées ci-dessus, et R"₂ et R"₃ représentent indiféremment l'un Z₄ ou Z₅ tels que définis ci-dessus et l'autre S-Z₂, tel que défini ci-dessus,
soit l'on soumet un composé de formule (XVI) : 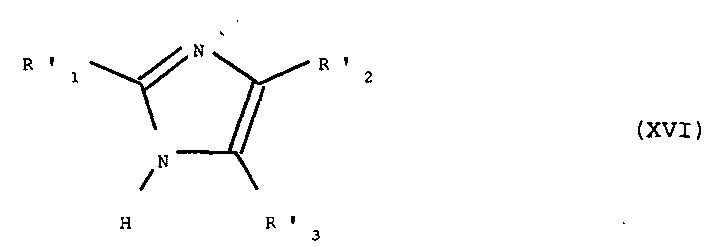 dans laquelle R'₁ a la signification indiquée ci-dessus et R'₂ et R'₃ ont les significations indiquées ci-dessus respectivement pour R₂ et R₃ dans lesquelles les éventuelles fonctions réactives sont éventuellement protégées par des groupements protecteurs, à une réaction avec le composé de formule (III) telle que définie ci-dessus, pour obtenir un produit de formule (I') : 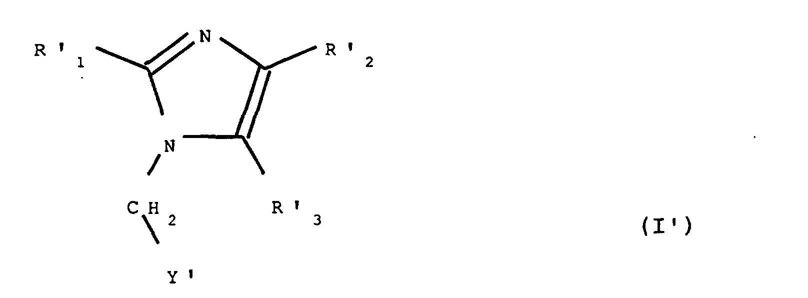 dans laquelle R'₁, R'₂, R'₃ et Y' ont les définitions indiquées ci-dessus,
produits de formules (I₁), (I₂), (I₃), (I₄), (XIV), (XV), (I₅) et (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction de transformation de fonction ester en fonction acyle,
d) une réaction de transformation de la fonction cyano en fonction acide,
e) une réaction de transformation de fonction acide en fonction amide, puis éventuellement en fonction thioamide,
f) une réaction de réduction de la fonction carboxy en fonction alcool,
g) une réaction de transformation de fonction alcoxy en fonction hydroxyle, ou encore de fonction hydroxyle en fonction alcoxy,
h) une réaction d'oxydation de fonction alcool en fonction aldéhyde, acide ou cétone,
i) une réaction de transformation du radical formyle en radical carbamoyle,
j) une réaction de transformation du radical carbamoyle en radical nitrile,
k) une réaction de transformation de radical nitrile en tétrazolyle,
l) une réaction d'oxydation de groupement alkylthio ou arylthio en sulfoxyde ou sulfone correspondant,
m) une réaction de transformation de fonction sulfure, sulfoxyde ou sulfone en fonction sulfoximine correspondante,
n) une réaction de transformation de fonction oxo en fonction thioxo,
o) une réaction de transformation du radical 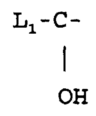 en radical 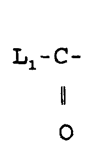 puis si nécessaire de nouveau en radical 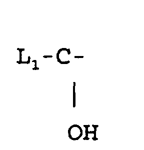 avec L₁ tel que défini ci-dessus,
p) une réaction de transformation de fonction acide en fonction 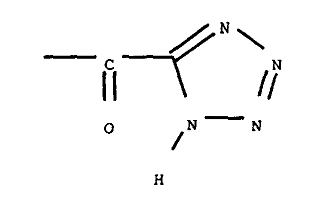
q) une réaction de transformation de la fonction β-cétosulfoxyde en fonction α-céto thio ester,
r) une réaction de transformation d'un carbamate en urée et notamment d'un sulfonylcarbamate en sulfonylurée,
s) une réaction d'élimination des groupements protecteurs que peuvent porter les fonctions réactives protégées,
t) une réaction de salification par un acide minéral ou organique ou par une base pour obtenir le sel correspondant,
u) une réaction de dédoublement des formes racémiques en produits dédoublés, lesdits produits de formule (I) ainsi obtenus étant sous toutes les formes isomères possibles racémiques, énantiomères et diastéréoisomères.

Dans des conditions préférentielles de mise en oeuvre de l'invention, le procédé décrit ci-dessus peut-être réalisé de la façon suivante. Dans le produit de formule (III), l'atome d'halogène représente de préférence un atome de brome mais peut également représenter un atome de chlore ou d'iode. La réaction de condensation des imidazoles de formules telles que définies ci-dessus (II), (VIII), (XVI), (XV) et (I₄) (dans le cas des produits de formules (XV) et (I₄), lorsque W représente P et après déprotection de l'atome d'azote), avec le composé de formule (III) telle que définie ci-dessus, pour obtenir respectivement les produits de formules (IV₁), (IX) lorsque W représente Y', (I₅) et (I') telles que définies ci-dessus peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

La réaction d'halogénation du composé de formule (IV₁) telle que définie ci-dessus en composé de formule (IV₂) telle que définie ci-dessus, peut être réalisée dans les conditions usuelles de l'homme du métier et notamment par bromation à l'aide de NBS dans CH₂Cl₂ ou encore Br₂ dans l'acide acétique.

Les composés de formules (IV₂), (IX) et (XIII) telles que définies ci-dessus peuvent être soumis à une réaction d'échange halogène métal sur l'atome d'halogène par réaction avec un composé organo métallique tel que le nBuli ou EtMgBr dans un solvant tel que le THF à une température d'environ
- 78°C pour le Buli et température ambiante pour EtMgBr.

Les réactions de carboxylation par CO₂ et de formylation par DMF des composés de formules (IV2) et (XIII) respectivement en composés de formules (I1) et (XIV) peuvent être réalisées selon les conditions usuelles de l'homme de métier soit par exemple dans le THF à température ambiante.

Z₁ et Z₂, identiques ou différents, représentent un radical alkyle, alkényle ou aryle de telle manière que Z₁-S-et Z₂-S- représentent les valeurs correspondantes définies ci-dessus par R₂ et R₃ dans lesquelles les éventuelles fonctions réactives sont éventuellement protégées par des groupements protecteurs.

L₁ et L_{1'}, identiques ou différents, représentent un radical alkyle, alkényle ou aryle de telle manière que R_{1"} représente les valeurs correspondantes choisies parmi les valeurs de R₁ telles que définies ci-dessus dans lesquelles les éventuelles fonctions réactives sont éventuellement protégées par des groupements protecteurs.

La réaction du composé de formule (IV₂) ou (XIII) telles que définies ci-dessus avec le composé de formule (VIₐ), (VI_{b}) ou (VI_{c}), telles que définies ci-dessus, pour obtenir le composé correspondant de formule respective (I₁) ou (XIV) telles que définies ci-dessus peut être réalisée d'une manière identique en utilisant EtMgBr comme agent de métallation dans le THF à température ambiante.

La réaction des composés de formules (IV₂) et (XIII) avec les composés de formule (Vₐ) ou (V_{b}) peuvent être réalisées selon les conditions usuelles de l'homme du métier soit par exemple dans le THF à température ambiante.

La réaction du composé de formule (IX) avec les composés de formules (X), (X'), (XI), (XII) et (XII') peut être réalisée selon les conditions usuelles de l'homme de métier soit par exemple dans le THF à température ambiante.

La fonction amine des composés de formules (XV) et (I₄) telle que définie ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, l'atome d'hydrogène peut être libéré dans du TFA ou encore en présence d'ion fluorure.

La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse ou encore de carbonate de cesium.

Les réactions de réduction ou oxydation des produits de formules (I₂) et (XV) respectivement en produits de formules (I₃) et (I₄) peuvent être réalisées selon les méthodes usuelles connues de l'homme du métier.

Selon les valeurs de R'₁, R"₁, R'₂, R"₂, R'₃, R"₃, les produits de formules (I₁), (I₂), (I₃), (I₄), (I₅), (XIV), (XV) et (I') constituent ou non des produits de formule (I) et peuvent donner des produits de formule (I), ou être transformés en d'autres produits de formule (I) en étant soumis à une ou plusieurs des réactions a) à u) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal
ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :

- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (I₁), (I₂), (I₃), (I₄), (I₅), (XIV), (XV) et (I'), telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) la réaction d'addition sur la fonction ester 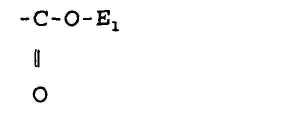 dans laquelle E₁ peut représenter un radical alkyle ounaryle éventuellement substitué et éventuellement protégé en fonction acyle 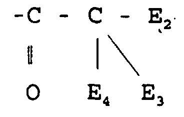 peut être réalisée notamment par action de l'anion carboné 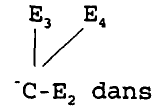 lequel E₂, E₃ et E₄, identiques ou différents sont choisis parmi l'atome d'hydrogène, les radicaux alkyl, alkylthioaryle, alkylsulfoxyde, arylsulfoxyde, alkylsulfone, arylsulfone, acyle, carboxy libre, salifié, estérifié ou amidifié, les radicaux alkyle, alkylthio et aryle étant éventuellement substitués et éventuellement protégés ainsi qu'il est indiqué ci-dessus.
   Une telle réaction est réalisée notamment ainsi qu'il est décrit dans la partie expérimentale, ou selon les méthodes usuelles connues de l'homme du métier.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) la réaction de transformation de fonction acide en fonction amide peut notamment être réalisée par formation d'abord d'un chlorure d'acide selon les conditions usuelles connues de l'homme du métier et par exemple par action de SOCl₂ puis amidification ci-dessus, ou encore par amidification directe de l'acide ci-dessus.
   Notamment on peut obtenir le radical 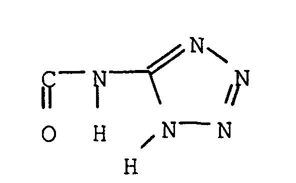 en transformant la fonction acide en chlorure d'acide, notamment par action de SOCl₂ dans un solvant tel que par exemple le toluène, ou le benzène, puis en faisant agir l'amine 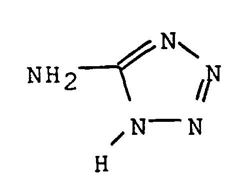
   L'amide ainsi obtenue peut alors si désiré, être transformée en thioamide par action notamment du réactif de LAWESSON dans le toluène,
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
i) j) Les réactions de transformation de radical formyle en radical carbamoyle et de radical carbamoyle en radical nitrile, sont réalisées notamment pour R₃ et R₄ selon les conditions usuelles connues de l'homme du métier, telles que par exemple passage par le céto nitrile et déplacement par une amine (Chem. Comm. 1971, p. 733).
k) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll.
l) Les éventuels groupements alkylthio ou arylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoique ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio ou arylthio et du réactif tel que notamment un peracide.
   L'obtention de la fonction sulfone peut être favorisée par un mélange du produit renfermant un groupement alkylthio
   ou arylthio avec un excés du réactif tel que notamment un peracide.
m) Les éventuelles fonctions sulfure, sulfoxyde ou sulfone des produits décrits ci-dessus peuvent être, si désiré, transformées en les fonctions sulfoximine correspondantes dans les conditions usuelles connues de l'homme du métier : des exemples non exhaustifs de préparation de produits renfermant une fonction sulfoximine sont décrites ci-dessous.
   Ainsi par exemple pour la préparation de composés tels que les N-(arylsulfonyl) sulfoximines et par exemple dans le cas où le groupement aryle que représente X' est un radical toluène, la sulfoximine peut être obtenue par action de l'azoture de paratoluènesulfonyle sur le sulfoxyde correspondant soit -S(O)CH₃ de préférence en présence de cuivre ainsi qu'il est indiqué, par exemple, dans la référence suivante : J. A. C. S., 95, pp. 4287 (1973) JOHNSON C.R. & coll.
   Une autre méthode également utilisée consiste à traiter la N-tosylsulfilimine, elle-même préparée à partir du sulfure par action, par exemple, de la chloramine "T", par un agent oxydant comme par exemple, l'hypochlorite de sodium dans des conditions de transfert de phase ainsi qu'il est indiqué, par exemple, dans la référence suivante :
   J. Org. Chem., 49, pp. 2282 (1984) AKUTAGAWA K.& coll.
n) la réaction de transformation de fonction oxo en fonction thioxo peut être réalisée notamment par le réactif de LAWESSON dans les conditions définies ci-dessus.
o) La réaction de transformation de radical 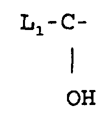 en radical 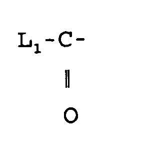 peut notamment être réalisée par l'oxyde de manganèse dans le dioxanne.
   La réaction inverse de transformation de radical 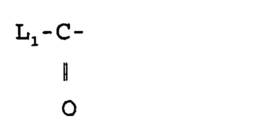 en radical 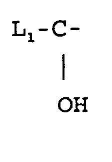 peut notamment être réalisée par le borohydrure de sodium dans l'éthanol.
p) La réaction de transformation de fonction acide en fonction tétrazolylcarboxy peut être réalisée par exemple par transformation préalable de la fonction acide en chlorure d'acide ainsi qu'il est indiqué ci-dessus, puis par action de Cu-C≡N, selon les conditions usuelles connues de l'homme du métier sur le chlorure d'acide ainsi obtenu, on obtient ainsi le radical 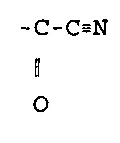 que l'on peut transformer en radical 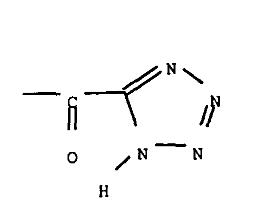 par exemple par action du composé Sn(Bu)₃N₃ dans le toluène,
q) la réaction de transformation de la fonction β céto sulfoxyde en fonction α céto thioester, peut être réalisée par bromation en α du cétosulfoxyde par exemple par action du NBS dans par exemple le chlorure de méthylène puis par une réaction de PUMMERER réalisée dans un mélange d'acide trifluoroacétique et de chlorure de méthylène ou encore un mélange d'acide sulfurique et de dioxanne.
   Notamment, ainsi qu'il est défini ci-dessus en c) et q), on peut réaliser le schéma réactionnel suivant : 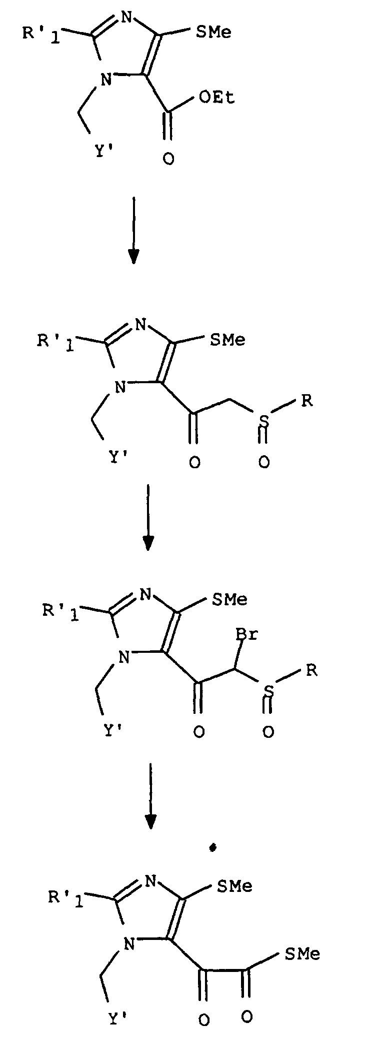 composés dans lesquels R'₁ et Y' ont les significations indiquées ci-dessus, et R représente un radical alkyle ou aryle éventuellement substitués ainsi qu'il est indiqué ci-dessus.
r) la réaction de transformation d'un carbamate en urée et notamment d'un sulfonylcarbamate en sulfonylurée, peut être réalisée par exemple au reflux d'un solvant comme par exemple le toluène en présence de l'amine adéquate.
s) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
t) Les produits décrits ci-dessus peuvent, si désiré, faire l'objet de réactions de salification par exemple par un acide minéral ou organique ou par une base minérale ou organique selon les méthodes usuelles connues de l'homme du métier.
u) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les composés de formule (I) tels que définis ci-dessus ainsi que leurs sels d'addition avec les acides présentent d'intéressantes propriétés pharmacologiques.

Les produits de formule (I) tels que définis ci-dessus, sont doués de propriétés antagonistes pour les récepteurs à l'endothéline et sont ainsi notamment inhibiteurs des effets de l'endothéline, notamment des effets vaso-constricteurs et hypertenseurs induits par l'endothéline. On note en particulier un effet antiischémique, l'activité vasoconstrictrice de l'endothéline étant abolie.

Les produits de formule I sont également capables de s'opposer aux effets stimulants de l'endothéline au niveau de tous les types cellulaires, notamment les cellules musculaires lisses, les cellules neuronales et les cellules osseuses.

Ces propriétés justifient leur application en thérapeutique et l'invention a également pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux et organiques ou avec les bases minérales et organiques pharmaceutiquement acceptables desdits produits de formule (I).

L'invention a particulièrement pour objet à titre de médicaments, les produits de formules (I_{A}) et (I_{B}) telles que définies ci-dessus, lesdits produits de formules (I_{A}) et (I_{B}) étant sous toutes les formes isoméres possibles racémiques ou optiquement actives, ainsi que les sels d'addition avec les acides minéraux ou organiques ou avec les bases minérales et organiques pharmaceutiquement acceptables desdits produits de formules (I_{A}) et (I_{B}).

L'invention a tout particulièrement pour objet à titre de médicaments, les produits de formule (I_{C}) telle que définie ci-dessus, lesdits produits de formule (I_{C}) étant sous toutes les formes isoméres possibles racémiques ou optiquement actives, ainsi que les sels d'addition avec les acides minéraux et organiques ou avec les bases minérales et organiques pharmaceutiquement acceptables desdits produits de formule (I).

L'invention a encore plus particulièrement pour objet à titre de médicaments, les produits de formule (I_{D}) telle que définie ci-dessus, lesdits produits de formule (I_{D}) étant sous toutes les formes isoméres possibles racémiques ou optiquement actives, ainsi que les sels d'addition avec les acides minéraux et organiques ou avec les bases minérales et organiques pharmaceutiquement acceptables desdits produits de formule (I).

L'invention a plus particulièrement pour objet, à titre de médicaments, les produits décrits ci-aprés dans les exemples et notamment les produits de formule (I) suivants :
- 1-((6-chloro-1,3-benzodioxol-5-yl) méthyl)-4-(((4-méthoxyphényl) méthyl) thio)-2-(phénylthio)-1H-imidazole-5-carboxaldéhyde
- acide 4-bromo-1-((6-chloro-1,3-benzodioxol-5-yl) méthyl)-2-phényl-1H-imidazole-5-carboxylique
- acide 1-((6-chloro-1,3-benzodioxol-5-yl) méthyl)-4-(((4-méthoxyphényl) méthyl) thio)-2-(phénylthio)-1H-imidazole-5-carboxylique
- acide 1-((6-chloro-1,3-benzodioxol-5-yl) méthyl)-4-(4-méthoxyphényl) thio)-2-(phénylthio)-1H-imidazole-5-carboxylique
- acide 1-((6-chloro-1,3-benzodioxol-5-yl) méthyl)-2,4-bis(4-(méthoxyphényl) thio)-1H-imidazole-5-carboxylique
- acide 1-((6-chloro 1,3-benzodioxol-5-yl) méthyl) 4-((3,4-diméthoxyphényl) thio) 2-(1,3-dioxolan-2-yl) 1H-imidazol 5-carboxylique
ainsi que leurs sels d'addition avec les acides minéraux ou organiques ou avec les bases minérales et organiques pharmaceutiquement acceptables.

Les médicaments, objet de l'invention, trouvent, par exemple, leur emploi dans le traitement de tous les spasmes vasculaires, dans le traitement des post-hémorragies cérébrales, dans le traitement des spasmes coronariens, des spasmes vasculaires périphériques ainsi que dans le traitement des insuffisances rénales. Ces médicaments peuvent également être utilisés dans le traitement de l'infarctus du myocarde, de l'insuffisance cardiaque congestive, dans la prévention des resténoses post-angioplastie, dans le traitement de l'athérosclérose et de certaines formes d'hypertension comme notamment l'hypertension pulmonaire, ainsi que dans le traitement de l'asthme.

Les médicaments, objet de l'invention, peuvent également trouver une application dans le traitement de l'ostéoporose, de l'hyperplasie prostatique et en tant que protecteurs neuronaux.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 300 mg par jour chez l'adulte, par voie orale ou de 1 à 100 mg par jour par voie intraveineuse.

Certains produits de départ de formules (II) et (XVI) sont connus et peuvent être préparés par exemple comme indiqué dans le brevet européen EP 0168950.

D'autres produits de départ de formules (II) et (XVI) peuvent en particulier être préparés comme indiqué dans le brevet européen EP 0465368.

Certains produits de départ de formules (II) et (XVI) sont commerciaux tels que par exemple le produit de formule (II) suivant :
- le 2-phénylimidazole par Aldrich.

Des exemples de produits commerciaux de formule (XVI) sont donnés dans les brevets EP 0465368 ou EP 0503162.

On peut encore notamment préparer certains produits de formules (II) et (XVI) à partir de produits de formule (II) par exemple en les soumettant à une ou plusieurs des réactions décrites ci-dessus en a) à u), réalisées dans les conditions également décrites ci-dessus.

Certains produits de formules (XVI) peuvent également être obtenus par monohalogénation du produit de formule (II) tel que défini ci-dessus en produit de formule (P₁) : 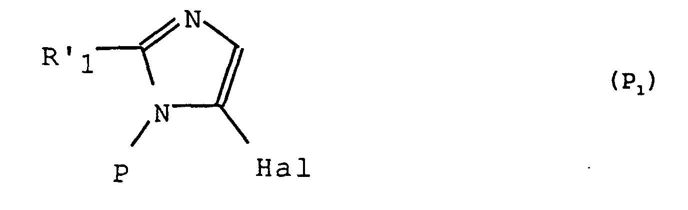 dans lequel R'₁ et P ont les significations indiquées ci-dessus pour le produit de formule (II), produit de formule (P₁) que l'on peut faire réagir, après échange suivant la réaction halogène métal connue de l'homme du métier, avec l'électrophile adapté, suivant les méthodes connues de l'homme du métier et notamment par exemple suivant le même type de réaction décrite ci-dessus pour passer par exemple du composé de formule (XIII) au composé de formule (XIV).

Les composés de départ de formule (VIII) peuvent être disponibles dans le commerce tel que le 2,4,5-tribromoimidazole chez Aldrich ou peuvent être préparés selon les méthodes usuelles connues de l'homme du métier.

Les produits de départ de formules (Vₐ), (V_{b}), (VIₐ), (VI_{b}) et (VI_{c}), sont commerciaux tels que notamment :
les produits de formule (Vₐ) ou (XI) suivants :
   - le sec-butyl disulfide
   - l'éthyl disulfide
   - l'isopropyl disulfide
   - le méthyl disulfide
   - le benzyl disulfide
   - le phényl disulfide
   - le propyl disulfide
les produits de formule (V_{b}) ou (XII) suivants :
   - le méthyl méthanethiosulfonate
   - le phényl benzenethiol sulfonate
les produits de formule (VIₐ) suivants :
   - le méthyl chloroformate
   - le benzyl chloroformate
   - l'isobutyl chloroformate
   - l'éthyl chloroformate
   - le N-propyl chloroformate
les produits de formule (VI_{b}) suivants :
   - le diméthyl carbonate
   - le diéthyl carbonate
les produits de formule (VI_{c}) suivants :
   - le di-tert-butyl oxalate
   - le diéthyl oxalate
   - le diméthyl oxalate

Les produits de départ de formules (X), (X') et (XII') sont commerciaux tels que notamment :
les produits de formule (X) suivants :
   - le benzaldehyde ou butanal
les produits de formule (X') suivants :
   - le chlorure de benzoyle ou de buturyle
les produits de formule (XII') suivants :
   - le chlorure de mesyle
   - le chlorure de tosyle

Un procédé de préparation de certains produits de formule (III) est notamment décrit dans le brevet européen EP 0465368.

Des exemples de préparation de composés de formule (III) sont également décrits dans la littérature et des exemples en sont donnés notamment dans le brevet US 4,880,804 ou par exemple dans la référence Chemistry and Industry 7 september 1987 HOWARD and COLQUHOUN pp. 612-617.

Notamment, le produit de formule (III) qui est le chlorure de 6-chloro pipéronyle est commercial chez Janssen.

La présente invention a enfin pour objet à titre de produits industriels nouveaux, les composés de formules (IV₁), (IV₂), (XIII), (XIV) et (XV) dans lesquels Y' représente le radical phényle éventuellement substitué par un ou plusieurs radicaux choisis parmi les atomes d'halogène et les radicaux dioxol, dans laquelle les éventuelles fonctions réactives sont éventuellement protégées par des groupements protecteurs.

L'invention a ainsi particulièrement pour objet l'utilisation des produits de formule (I) telle que définie ci-dessus, pour la préparation de compositions pharmaceutiques destinées au traitement d'affections résultant d'une stimulation anormale des récepteurs de l'endothéline.

L'invention a plus particulièrement pour objet l'utilisation des produits de formule (I) telle que définie ci-dessus, pour la préparation de compositions pharmaceutiques destinées au traitement de l'hypertension induite par l'endothéline.

L'invention a tout particulièrement pour objet l'utilisation des produits de formule (I) telle que définie ci-dessus, pour la préparation de compositions pharmaceutiques destinées au traitement de tous les spasmes vasculaires et du traitement des post-hémorragies cérébrales et des insuffisances rénales.

L'invention a également pour objet l'utilisation des produits de formule (I) telle que définie ci-dessus, pour la préparation de compositions pharmaceutiques destinées au traitement de l'infarctus du myocarde et à la prévention des restéroses post-angioplastie.

Les exemples suivants illustrent l'invention sans toutefois la limiter.

### EXEMPLE 1 : 4-bromo-1-((6-chloro-1,3-benzodioxol-5-yl) méthyl)-2-(phénylthio)-1H-imidazole-5-carboxaldehyde

### STADE 1 : 1-((6-chloro-1,3-benzodioxol-5-yl)méthyl)-2,4,5-tribromo-1H-imidazole

On introduit 25 g de 2,4,5 tribromoimidazole dans 500 ml de diméthylformamide et ajoute 4,3 g d'hydrure de sodium. L'agitation est maintenue 10 mn à température ambiante. On ajoute ensuite au milieu réactionnel 18,4 g de chlorure de 6 chloro pipéronyl, puis 25 g d'iodure de sodium et l'agitation est poursuivie pendant 15 mn à température ambiante.

Le milieu réactionnel est finalement versé sur 3 1 d'eau, on essore, lave abondamment par de l'eau, puis successivement par 250 ml d'éthanol, 250 ml d'isopropanol, puis finalement par 250 ml d'éther isopropylique.

On sèche et récupère 31.5 g de produit attendu (solide crème) Pf = 225°C.
IR CHC13 (cm⁻¹)
Absence de =C-NH
Hétérocycle aromatique 1624-1506-1497-1485

### STADE 2 : 1-((6-chloro-1,3-benzodioxol-5-yl)méthyl)-4,5-dibromo-2-(phénylthio)-1H-imidazole

On introduit 4,73 g du produit obtenu ci-dessus au stade 1 dans 100 ml de mélange chlorure de méthylène/éther sulfurique (20/80), à laquelle on ajoute goutte à goutte 3,5 ml de bromure d'éthyle magnésium : on maintient sous agitation pendant 30 mn à température ambiante.

On introduit alors au milieu réactionnel obtenu 2,9 g de phényl benzène thiosulfonate. L'agitation est maintenue pendant 2 h à température ambiante.

On hydrolyse le milieu par addition d'acide chlorhydrique dilué, après extraction par de l'acétate d'éthyle, lavage abondant à l'eau puis séchage, on récupère un solide crème que l'on purifie par empatage dans 50 ml d'éthanol. On obtient ainsi 3,35 g du produit attendu Pf 165°C.
IR CHC13 (cm⁻¹)
Hétérocycle + aromatique 1585-1508-1484
Micro analyse

| | % calculés | % trouvés |
|---|---|---|
| C | 40,6 | 40,7 |
| H | 2,2 | 2,1 |
| N | 5,57 | 5,4 |
| S | 6,37 | 6,4 |
| Br | 31,8 | 31,4 |
| Cl | 7,05 | 6,8 |

### STADE 3 : 4-bromo-1-((6-chloro-1,3-benzodioxol-5-yl)méthyl)-2-(phénylthio)-1H-imidazole-5-carboxaldehyde

On introduit 6,4 g du produit obtenu au stade 2 ci-dessus dans 100 ml de tétrahydrofuranne et on ajoute goutte à goutte 9,3 ml de bromure d'éthyle magnésien. On maintient sous agitation pendant 30 mn à température ambiante.

On introduit alors au milieu réactionnel obtenu 5 équivalents de diméthyl formamide.

L'agitation est maintenue pendant 2 h à température ambiante.

On hydrolyse le milieu par addition d'acide chlorhydrique dilué, après extraction de l'acétate d'éthyle, lavage abondant à l'eau puis séchage, on récupère un solide marron que l'on purifie par chromatographie sur silice avec pour éluant du chlorure de méthylène. On obtient 5 g de produit attendu (Pf = 155°C) dont 500 mg sont recristallisés dans 25 ml d'éthanol : après essorage puis séchage on récupère 420 mg de produit attendu (solide blanc) (pf = 155°C).
IR CHC13 (cm⁻¹)
C=0 1669
Aromatique+Hétéroatome 1627-1580-1505-1485
Micro analyse

| ) | % calculés | % trouvés |
|---|---|---|
| C | 47,9 | 47,9 |
| H | 2,67 | 2,6 |
| N | 6,2 | 6,1 |
| S | 7,09 | 7,2 |
| Br | 17,68 | 17,7 |
| Cl | 7,84 | 7,7 |

### EXEMPLE 2 : 1-((6-chloro-1,3-benzodioxol-5-yl)méthyl)-4-(((4-méthoxyphényl)méthyl)thio)-2-(phénylthio)-1H-imidazole-5-carboxaldehyde

On introduit 620 mg d'hydrure de sodium 50 % dans l'huile dans 50 ml de tétrahydrofuranne, à laquelle on ajoute lentement 1,8 ml de 4-méthoxy-benzeneméthanethiol. L'agitation est maintenue pendant 15 mn à température ambiante.

On introduit ensuite au thiolate ainsi formé une solution de 3,8 g du produit de l'exemple 1 dans 25 ml de tétrahydrofuranne. L'agitation est poursuivie dans ces conditions pendant 2 h 30mn.

Le milieu réactionnel est finalement versé sur de la soude 0,1N, après extraction par de l'acétate d'éthyle, lavage abondant à l'eau puis séchage, on récupère une résine brune que l'on purifie par chromatographie sur silice avec pour éluant du chlorure de méthylène 80/cyclohexane 20 puis du chlorure de méthylène + 20 % d'acétate d'éthyle.

On recueille 2 fractions 1,97 g du produit attendu (Pf = 135°C) et 1,98 g d'un second produit qui se forme dans les mêmes conditions opératoires : le 1-((6-chloro-1,3-benzodioxol-5-yl)méthyl)-2,4-bis(((4-méthoxyphényl)méthyl) thio)-1H-imidazole-5-carboxaldehyde
CONTROLES POUR A
IR CHC13 (cm⁻¹)
C=O 1660
aromatique + Hétérocycle 1611-1580-1513-1505-1485
Micro analyse

| | % calculés | % trouvés |
|---|---|---|
| C | 59,47 | 59,2 |
| H | 4,03 | 3,8 |
| N | 5,33 | 5,2 |
| S | 12,21 | 12,0 |
| Cl | 6,75 | 6,7 |

CONTROLES POUR B
IR CHC13 (cm⁻¹)
absence de S phényl
C=O 1655
aromatique Hétérocycle 1612-1585-1512-1508-1485

### EXEMPLE 3 : 4-bromo-1-((6-chloro-1,3-benzodioxol-5-yl) méthyl)-1H-imidazole-5-carboxaldehyde

### STADE 1 : 1-((6-chloro-1,3-benzodioxol-5-yl)méthyl)-4,5-dibromo-1H-imidazole

On procède comme au stade 2 de l'exemple 1 à partir de 6 g du produit obtenu au stade 1 de l'exemple 1 dans 300 ml de mélange chlorure de méthylène/éther sulfurique (60/240), à laquelle on ajoute goutte à goutte 4,6 ml de bromure d'éthyle magnésium puis maintient sous agitation pendant 30 mn à température ambiante et introduit alors au milieu réactionnel obtenu 100 ml d'eau glacée.

Après acidification, extraction, lavage puis séchage, on récupère 3,35 g de produit attendu (solide crème homogène) (Pf 150°C).
IR CHC13 (cm⁻¹)
Hétérocycle + aromatique 1626-1508-1484

### STADE 2 : 4-bromo-1-((6-chloro-1,3-benzodioxol-5-yl)méthyl)-1H-imidazole-5-carboxaldehyde

On procède comme au stade 3 de l'exemple 1 à partir de 900 mg de produit obtenu au stade 1 ci-dessus dans 10 ml de tétrahydrofuranne et on ajoute goutte à goutte 1,7 ml de bromure d'éthyle magnésien. On maintient sous agitation pendant 15 mn à température ambiante.

On introduit alors au milieu réactionnel obtenu 0,9 ml soit 5 équivalents de diméthyl formamide. L'agitation est maintenue pendant 2h à température ambiante.

Après hydrolyse du milieu par addition d'acide chlorhydrique dilué, extraction, lavage puis séchage, on récupère un solide crème que l'on purifie par chromatographie sur silice avec pour éluant du chlorure de méthylène + 5 % d'acétate d'éthyle. On obtient ainsi 630 mg de produit attendu (Pf = 136°C).
IR CHC13 (cm⁻¹)
C=O 1670
Aromatique + Hétéroatome 1625-1510-1485

### EXEMPLE 4 : 1-((6-chloro-1,3-benzodioxol-5-yl)méthyl)-4-(((4-méthoxyphényl)méthyl)thio)-1H-imidazole-5-carboxaldehyde

On procède comme à l'exemple 2 à partir de 90 mg d'hydrure de sodium 50 % dans l'huile dans 10 ml de tétrahydrofuranne, à laquelle on ajoute lentement 0,26 ml de 4-méthoxy-benzeneméthanethiol.

Puis on introduit au thiolate ainsi formé une solution de 580 mg du produit de l'exemple 3 dans 5 ml de tétrahydrofuranne et l'agitation est poursuivie dans ces conditions pendant 2h30mn. En procédant comme à l'exemple 2, on récupère une résine brune que l'on purifie par chromatographie sur silice avec pour éluant du chlorure de méthylène 95/acétate d'éthyle 5 et obtient 400 mg de produit purifié par recristallisation dans 20 ml d'éthanol (Pf = 160°C).
IR CHCl3 (cm⁻¹)
C=O 1664
aromatique + hétérocycle 1610-1584-1513-1506-1483 Micro analyse

| | % calculés | % trouvés |
|---|---|---|
| C | 57,62 | 57,2 |
| H | 4,11 | 4,0 |
| N | 6,71 | 6,7 |
| S | 7,69 | 7,7 |
| Cl | 8,5 | 8,7 |

### EXEMPLE 5 : acide 4-bromo-1-((6-chloro-1,3-benzodioxol-5 yl)méthyl)-2-phényl-1H-imidazole-5-carboxylique

### STADE 1 : 1-((6-chloro-1,3-benzodioxol-5-yl)méthyl)-2-phényl-1H-imidazole

On dissout 1,44 g de 2-phényl imidazole dans 30 ml de diméthylformamide, on ajoute par petites portions 550 mg d'hydrure de sodium à 50 % dans l'huile, laisse 15 minutes à température ambiante puis ajoute 2,05 g de chlorure de 6 chloro pipéronyle et laisse une nuit à température ambiante. On évapore le diméthylformamide, on ajoute 50 ml de chlorure d'ammonium saturé et on extrait avec 3 fois 70 ml de chlorure de méthylène, sèche, évapore et chromatographie sur silice avec pour éluant l'acétate d'éthyle. On obtient ainsi 2,8 g de produit attendu Pf = 163°C.

### STADE 2 : 1-((6-chloro-1,3-benzodioxol-5-yl)méthyl)4,5-di-bromo-2-phényl-1H-imidazole

On dissout 2,8 g du produit obtenu au stade 1 ci-dessus dans 50 ml de chlorure de méthylène puis ajoute 3,6 g de N-Bromo-succinimide par petites portions et laisse une nuit à température ambiante. On lave alors la phase organique avec de la soude N puis une solution saturée de chlorure de sodium, sèche, évapore et on récupère 3,3 g de produit attendu après empatage dans l'éther Pf = 173°C.

### STADE 3 : acide 4-bromo-1-((6-chloro-1,3-benzodioxol-5-yl) méthyl)-2-phényl-1H-imidazole-5-carboxylique

A une solution de 706 mg du produit obtenu au stade 2 ci-dessus dans 15 ml de tétrahydrofuranne anhydre, on ajoute goutte à goutte, à température ambiante, 750 µl d'une solution 3M de bromure d'éthyle magnésien dans l'éther, on laisse 30 minutes à température ambiante puis on fait buller dans la solution un excès de CO2 pendant 30 minutes. On hydrolyse avec chlorure d'ammonium saturé puis on acidifie à pH1 avec bromure d'éthyle magnésien 2N et on extrait à l'acétate d'éthyle. On sèche, évapore, recristallise le résidu dans le chlorure de méthylène pour obtenir 310 mg de produit attendu Pf = 215°C.

### EXEMPLE 6 : 4-bromo 1-((6-chloro-1,3-benzodioxol-5-yl) méthyl)- 2-phényl-1H-imidazole-5-carboxaldehyde

A une solution de 3,7 g du produit obtenu au stade 2 de l'exemple 5 dans 80 ml de tétrahydrofuranne anhydre, on ajoute goutte à goutte 5,2 ml de bromure d'éthyle magnésien, 3M dans l'éther, laisse 20 minutes à température ambiante puis ajoute 5 ml de diméthylformamide anhydre et laisse encore 2 heures à température ambiante. On hydrolyse avec chlorure d'ammonium saturé et on extrait à l'acétate d'éthyle. On sèche, évapore, empate le résidu solide à l'éther et obtient 2,5 g de produit attendu Pf = 148°C.

### EXEMPLE 7 : 1-((6-chloro-1,3-benzodioxol-5-yl)méthyl)-4-(((4-méthoxyphényl)méthyl)thio)-2-phényl-1H-imidazole-5-carboxaldehyde

On procède comme à l'exemple 2 à partir du produit de l'exemple 6.

A une solution de 635 µl de paraméthoxybenzylthiol dans 50 ml de diméthyformamide on ajoute 250 mg de hydrure de sodium à 50 % dans l'huile, laisse 15 minutes à température ambiante et ajoute 1,7 g du produit de l'exemple 6 puis laisse 4 heures à température ambiante et évapore le diméthylformamide. On reprend le résidu dans la soude 1N et extrait au chlorure de méthylène. On sèche la phase organique, évapore et chromatographie sur silice avec pour éluant acétate d'éthyle/cyclohexane 2/8. On obtient 1,31 g de produit attendu Pf = 120°C.

### EXEMPLE 8 : 1-((6-chloro-1,3-benzodioxol-5-yl)méthyl)-4-(((4-méthoxyphényl)méthyl)thio)-2-phényl-1H-imidazole-5-carboxylate de méthyle

On procède comme à l'exemple 9 à partir du produit de l'exemple 7.

A une solution de 600 mg du produit de l'exemple 7 dans le chlorure de méthylène, on ajoute respectivement 30 ml de méthanol, 120 µl d'acide acétique, 2,25 g d'oxyde de manganèse et 300 mg de cyanure de sodium. On laisse agiter à température ambiante 96 heures, filtre, lave à l'acétate d'éthyle et évapore. On obtient 410 mg de produit attendu Pf = 158°C.

### EXEMPLE 9 : 1-((6-chloro-1,3-benzodioxol-5-yl)méthyl)-4-(((4-méthoxyphényl)méthyl)thio)-2(phénylthio)-1H-imidazole-5-carboxylate de méthyle

On introduit 970 mg du produit de l'exemple 2 dans un mélange de chlorure de méthylène/méthanol (25 ml/100 ml) et ajoute ensuite successivement 5 g d'oxyde de manganèse, 500 mg de cyanure de sodium et 200 µl d'acide acétique. L'agitation est alors maintenue pendant 72 h à température ambiante.

On essore, lave par du chlorure de méthylène puis concentre sous vide.

Le produit brut est purifié par passage rapide sur silice avec pour éluant du chlorure de méthylène.

On obtient 910 mg de produit attendu brut (Pf = 130°C) que l'on recristallise dans 100 ml d'éthanol. Après essorage puis séchage, on récupère 815 mg de produit attendu (solide blanc) (Pf = 130°C).
IR CHC13 (cm⁻¹) ester 1699 1436
aromatique + hétérocycle 1610-1584-1513-1505-1483
Micro analyse

| | % calculés | % trouvés |
|---|---|---|
| C | 58,4 | 58,5 |
| H | 4,17 | 4,1 |
| N | 5,04 | 4,9 |
| S | 11,55 | 11,4 |
| Cl | 6,38 | 6,6 |

### EXEMPLE 10 : acide 1-((6-chloro-1,3-benzodioxol-5-yl)méthyl)-4-(((4-méthoxyphényl)méthyl)thio)-2-(phénylthio)-1H-imidazole-5-carboxylique

On introduit 500 mg du produit obtenu à l'exemple 9 dans le mélange éthanol/tétrahydrofuranne (15 ml/20 ml) et ajoute 15 ml de soude 2N. L'agitation est maintenue pendant 48h à température ambiante.

Le milieu réactionnel est alors acidifié par addition d'acide chlorhydrique 2N et après extraction par de l'acétate d'éthyle, lavage abondant à l'eau puis séchage, on récupère 340 mg d'un résidu crème que l'on purifie par chromatographie sur silice avec pour éluant acétate d'éthyle 50/chlorure de méthylène 50.

On purifie par recristallisation dans 20 ml d'éthanol et après essorage puis séchage, on récupère 180 mg de produit attendu (solide blanc) (Pf = 180°C)
IR CHC13 (cm⁻¹)
-OH 3510
-C=0 1704 1659
Système conjugué 1610-1580-1513-1505-1483
Micro analyse

| | % calculés | % trouvés |
|---|---|---|
| C | 57,7 | 57,5 |
| H | 3,91 | 3,9 |
| N | 5,18 | 5,2 |
| S | 11,85 | 11,8 |
| Cl | 6,55 | 6,7 |

### EXEMPLE 11 : 1-((6-chloro-1,3-benzodioxol-5-yl)méthyl)-4-(((4-méthoxyphényl)méthyl)thio)-1H-imidazole-5-carboxylate de méthyle

On procède comme à l'exemple 9 à partir de 1 g du produit de l'exemple 4 dans un mélange chlorure de méthylène/méthanol (20 ml/50 ml) et ajoute ensuite successivement 6 g d'oxyde de manganèse, 750 mg de cyanure de sodium et 400 µl d'acide acétique. L'agitation est alors maintenue pendant 48h à température ambiante.

Procédant comme à l'exemple 9, on obtient 700 mg de produit attendu Pf = 123°C
IR CHC13 (cm⁻¹)
ester 1700
aromatique + hétérocycle 1611-1580-1513-1505-1483

### EXEMPLE 12 : acide 1-((6-chloro-1,3-benzodioxol-5-yl)méthyl)-4-(((4-méthoxyphényl)méthyl)thio)-1H-imidazole-5-carboxylique

On procède comme à l'exemple 10 à partir de 600 mg du produit de l'exemple 11 dans un mélange éthanol/tétrahydrofuranne (25 ml/30 ml) et ajoute 50 ml de soude N.

L'agitation est maintenue pendant 48h à température ambiante.

Procédant comme à l'exemple 10, on obtient ainsi 310 mg de produit attendu (Pf = 195°C)
IR NUJOL (cm⁻¹)
Absorption générale OH/NH
-C=O 1690
aromatique + hétérocycle 1610-1583-1513-1508-1488
Micro analyse

| | % calculés | % trouvés |
|---|---|---|
| C | 55, 5 | 55,4 |
| H | 3,95 | 3,7 |
| N | 6,47 | 6,5 |
| S | 7,4 | 7,3 |
| Cl | 8,2 | 8,2 |

### EXEMPLE 13 : 1-((6-chloro-1,3-benzodioxol-5-yl)méthyl)-4-(((4-méthoxyphényl)thio)-2-(phénylthio)-1H-imidazole-5-carboxaldehyde

On procède comme à l'exemple 2 à partir de 550 mg d'hydrure de sodium 50 % dans l'huile dans 50 ml de tétrahydrofuranne, puis ajoute 1,4 ml de 4-méthoxy-benzènethiol. L'agitation est maintenue pendant 15 mn à température ambiante.

On introduit ensuite au thiolate ainsi formé une solution de 3,7 g du produit de l'exemple 1 dans 50 ml de tétrahydrofuranne. L'agitation est poursuivie dans ces conditions pendant 1h 30mn.

Procédant comme à l'exemple 2, on recueille 2 fractions : 3,02 g du produit attendu et 600 mg du produit de l'exemple 16.
IR CHC13 (cm⁻¹)
C=O 1663
aromatique + hétérocycle 1593-1580-1570-1505-1494-1484 dont S-Φ-OCH3

### EXEMPLE 14 : 1-((6-chloro-1,3-benzodioxol-5-yl)méthyl)-4-(((4-méthoxyphényl)thio)-2-(phénylthio)-1H-imidazole-5-carboxylate de méthyle

On procède comme à l'exemple 9 à partir de 1 g du produit de l'exemple 13 dans le mélange chlorure de méthylène/méthanol (20 ml/100 ml) et ajoute ensuite successivement 5 g d'oxyde de manganèse, 500 mg de cyanure de sodium et 300 µl d'acide acétique.

L'agitation est alors maintenue pendant 24h à température ambiante.

En procédant comme à l'exemple 9, on obtient 820 mg de produit attendu (Pf = 140°C).
IR CHCL3 (cm⁻¹)
ester 1704
aromatique + hétérocycle 1593-1575-1505-1494-1483

### EXEMPLE 15 : Acide 1-((6-chloro-1,3-benzodioxol-5-yl)méthyl)-4-(4-méthoxyphényl)thio)-2-(phénylthio)-1H-imidazole-5-carboxylique

On procède comme à l'exemple 10 à partir de 780 mg du produit de l'exemple 14 dans un mélange éthanol/tétrahydrofuranne 20 ml/ 30 ml et ajoute 20 ml de soude N.

L'agitation est maintenue pendant 4h30mn à température ambiante.

Procédant comme à l'exemple 10, on obtient 550 mg de produit attendu (Pf = 170°C).
IR CHC13 (cm⁻¹)
-OH/NH complexe
C=O 1686
Aromatique + Système conjugué 1592-1573-1503-1493
Micro analyse

| | % calculés | % trouvés |
|---|---|---|
| C | 57,0 | 56,8 |
| H | 3,63 | 3,4 |
| N | 5,3 | 5,3 |
| S | 12,16 | 11,8 |
| Cl | 6,72 | 6,8 |

### EXEMPLE 16 : 1-((6-chloro-1,3-benzodioxol-5-yl)méthyl)-2,4-bis((4-méthoxyphényl)thio))-1H-imidazole-5-carboxaldehyde

Le produit de l'exemple 16 (600 mg) est obtenu lors de la préparation de l'exemple 13 décrite ci-dessus.
IR CHC13 (cm⁻¹)
C=O 1663
Aromatique + hétérocycle 1593-1575-1505-1495-1484-dont S-Φ-OCH3

### EXEMPLE 17 : 1-((6-chloro-1,3-benzodioxol-5-yl)méthyl)-2,4-bis((4-méthoxyphényl)thio))-1H-imidazole-5-carboxylate de méthyle

On procède comme à l'exemple 9 à partir de 470 mg du produit de l'exemple 16 dans un mélange chlorure de méthylène/méthanol 10 ml/50 ml et ajoute ensuite successivement 2,5 g d'oxyde de manganèse, 250 mg de cyanure de sodium et 200 µl d'acide acétique. L'agitation est alors maintenue pendant 24 h à température ambiante.

Procédant comme à l'exemple 9, on obtient 315 mg du produit attendu (Pf = 130°C).
IR CHCL3 (cm⁻¹)
ester 1702
Aromatique + hétérocycle 1593-1575-1505-1494-1483

### EXEMPLE 18 : Acide 1-((6-chloro-1,3-benzodioxol-5-yl)méthyl)-2,4-bis(4-(méthoxyphényl)thio)-1H-imidazole-5-carboxylique

On procède comme à l'exemple 10 à partir de 280 mg du produit de l'exemple 17 dans un mélange éthanol/tétrahydrofuranne 20 ml/ 20 ml à laquelle on ajoute 20 ml de soude N. L'agitation est maintenue pendant 5h à température ambiante.

Procédant comme à l'exemple 10, on obtient 240 mg de produit attendu (Pf = 110°C)
IR NUJOL (cm⁻¹)
-C=O 1648
Aromatique + Hétéroatome 1593-1573-1505-1489
Micro analyse

| | % calculés | % trouvés |
|---|---|---|
| C | 56,06 | 55,7 |
| H | 3,8 | 3,8 |
| N | 5,02 | 4,7 |
| S | 11,5 | 11,2 |
| Cl | 6,36 | 6,5 |

### EXEMPLE 19 : 1-((6-chloro-1,3-benzodioxol-5-yl)méthyl)-5-(hydroxyméthyl)-4-(4-(méthoxyphényl)thio)-2-(phénylthio)-1H-imidazole

On introduit 820 mg du produit de l'exemple 13 dans 50 ml d'éthanol et ajoute 60 mg de borohydrure de sodium.

L'agitation est maintenue pendant 30 mn à température ambiante.

On ajoute 0,5 ml d'acide acétique puis 50 ml d'eau glacée.

Après 10 mn d'agitation, on essore lave par de l'eau, sèche et récupère 700 mg de produit brut que l'on purifie par recristallisation dans 20 ml d'isopropanol, essore et lave par 20 ml d'éthanol.

On obtient 410 mg de produit attendu (Pf = 146°C)
IR CHC13 (cm⁻¹)
-OH 3600 + associé
Système conjugué 1627-1594-1583-1575-1505-1494-1484 Micro analyse

| | % calculés | % trouvés |
|---|---|---|
| C | 58,5 | 58,2 |
| H | 4,13 | 4,0 |
| N | 5,46 | 5,4 |
| Cl | 6,91 | 7,0 |
| S | 12,5 | 12,3 |

### EXEMPLE 20 : 1-((6-chloro-1,3-benzodioxol-5-yl)méthyl)-4-((4-méthoxyphényl)thio)-2-phényl-1H-imidazole-5-carboxaldehyde

On procède comme à l'exemple 2 à partir de 4 g du produit de l'exemple 6 en utilisant 640 mg d'hydrure de sodium à 50 % dans l'huile et 1,64 ml de 4-méthoxy thiophénol et obtient ainsi 4,05 g de produit attendu (Pf = 116°C)

### EXEMPLE 21 : 1-((6-chloro-1,3-benzodioxol-5-yl)méthyl)-4-((4-méthoxyphényl)thio)-2-phényl-1H-imidazole-5-carboxylate de méthyle

On procède comme à l'exemple 9 à partir de 2 g du produit de l'exemple 20 en utilisant 10 g d'oxyde de manganèse, 1 g de cyanure de sodium, 200 ml de méthanol, 40 ml de chlorure de méthylène et 600 µl d'acide acétique, on obtient ainsi 1,64 g de produit attendu (Pf = 161°C)

### EXEMPLE 22 : Acide 1-((6-chloro-1,3-benzodioxol-5-yl)méthyl)-4-(4-méthoxyphényl)thio)-2-phényl-1H-imidazole-5-carboxylique

On procède comme à l'exemple 10 à partir de 1 g du produit de l'exemple 21 en utilisant 25 ml de soudelN, 25 ml d'éthanol et 50 ml de tétrahydrofuranne. On obtient ainsi 692 mg de produit attendu (Pf = 190°C)

### EXEMPLE 23 : 2-benzoyl-1-((6-chloro-1,3-benzodioxol-5-yl) methyl)-4-((4-méthoxyphényl)thio)-1H-imidazole-5-carboxylate de méthyle

### STADE 1 : 1-((6-chloro-1,3-benzodioxol-5-yl)methyl)-4,5-dibromo-α-phényl-1H-imidazole-2-methanol

On procède comme au stade 2 de l'exemple 1 à partir de 9,5 g de produit obtenu au stade 1 de l'exemple 1 dans un mélange de chlorure de méthylène/éther sulfurique 100ml/350ml et ajoute goutte à goutte 7,3 ml de bromure d'éthyle magnésium. On maintient sous agitation pendant 20 mn à température ambiante.

On introduit alors au milieu réactionnel obtenu 10 ml de benzaldehyde.

L'agitation est maintenue pendant 2h à température ambiante.

Procédant comme au stade 2 de l'exemple 1, on obtient 5,34 g de produit attendu.
IR CHC13 (cm⁻¹)
-OH 3603 + absorption générale
Aromatique hétéroatome 1627-1603-1505-1484

### STADE 2 : 4-bromo-1-((6-chloro-1,3-benzodioxol-5-yl)méthyl)-2-(α-hydroxy-(phénylméthyl))-1H-imidazole-5-carboxaldehyde

On procède comme au stade 3 de l'exemple 1 à partir de 5,3 g du produit obtenu au stade 1 ci-dessus dans 100 ml de tétrahydrofuranne et ajoute goutte à goutte 11 ml de bromure d'éthyle magnésien. On maintient sous agitation pendant 15 mn à température ambiante.

On introduit alors au milieu réactionnel obtenu 5 ml de diméthylformamide. L'agitation est maintenue pendant 2h à température ambiante.

Procédant comme au stade 3 de l'exemple 1, on obtient 2,067 g de produit attendu.
IR CHC13 (cm⁻¹)
C=O 1674
-OH 3596 complexe
Aromatique + hétérocycle 1628-1602-1505-1485

### STADE 3 : 1-((6-chloro-1,3-benzodioxol-5-yl)méthyl)-2-(α-hydroxy-(phénylméthyl))-4-((4-méthoxyphényl)thio)-1H-imidazole-5-carboxaldehyde

On procède comme à l'exemple 2 à partir d'une suspension de 390 mg d'hydrure de sodium 50 % dans l'huile dans 50 ml de tétrahydrofuranne, à laquelle on ajoute lentement 1 ml de 4-méthoxy-benzenethiol. L'agitation est maintenue pendant 15 mn à température ambiante.

On ajoute ensuite au thiolate ainsi formé une solution de 2,6 g du produit obtenu au stade 2 ci-dessus dans 25 ml de tétrahydrofuranne. L'agitation est poursuivie dans ces conditions pendant 2h.

Procédant comme à l'exemple 2, on obtient 2,15 g de produit attendu.
IR CHC13 (cm⁻¹)
C=O 1667
-OH 3598 associé
aromatique + hétérocycle 1593-1574-1505-1494-1484

### STADE 4 : 2-benzoyl-1-((6-chloro-1,3-benzodioxol-5-yl) methyl)-4-((4-méthoxyphényl)thio)-1H-imidazole-5-carboxylate de méthyle

On procède comme à l'exemple 9 à partir de 2,15 g du produit obtenu au stade 3 ci-dessus dans 200 ml de méthanol, on ajoute ensuite successivement 11 g d'oxyde de manganèse, 1,1 g de cyanure de sodium et 700 µl d'acide acétique.

L'agitation est alors maintenue pendant 48h à température ambiante.

Procédant comme à l'exemple 9, on obtient 1,32 g de produit attendu (Pf = 166°C)
IR CHC13 (cm⁻¹)
Absence d'OH
C=O 1716-1653
Aromatique + hétérocycle 1597-1579-1508-1495

### EXEMPLE 24 : Acide 2-benzoyl-1-((6-chloro-1,3-benzodioxol-5-yl)méthyl)-4-((4-méthoxyphényl)thio-1H-imidazole-5-carboxylique

On procède comme à l'exemple 10 à partir de 400 mg du produit de l'exemple 23 dans un mélange éthanol/tétrahydrofuranne 30 ml/ 30 ml et ajoute 10 ml de soude N.

L'agitation est maintenue pendant 48h à température ambiante.

Procédant comme à l'exemple 10, on obtient 385 mg de produit attendu (Pf = 210°C)
IR NUJOL (cm⁻¹)
Absorption générale OH/NH
-C=O 1679-1652
Aromatique + hétérocycle 1590-1572-1503-1485
Micro analyse

| | % calculés | % trouvés |
|---|---|---|
| C | 59,71 | 59,4 |
| H | 3,66 | 3,7 |
| N | 5,35 | 5,2 |
| S | 6,13 | 6,0 |
| Cl | 6,77 | 7,0 |

### EXEMPLE 25 : Acide 1-((6-chloro-1,3-benzodioxol-5-yl)méthyl)-4-(((4-méthoxyphényl)méthyl)thio)-2-phényl-1H-imidazole-5-carboxylique

On procède comme à l'exemple 10 à partir du produit de l'exemple 8.

A une solution de 740 mg du produit de l'exemple 8 dans un mélange tétrahydrofuranne/acétate d'éthyle (50 ml/50ml) on ajoute 30 ml de soude 2N et laisse 96 h à température ambiante. On évapore, reprend avec 30 ml d'eau, acidifie avec de l'acide chlorhydrique 1N et filtre.

On obtient ainsi 440 mg de produit attendu (Pf = 190°C).

### EXEMPLE 26 de composition pharmaceutique.

On a préparé des comprimés répondant à la formule suivante :

| | |
|---|---|
| Produit de l'exemple 10 | 50 mg |
| Excipient pour un comprimé terminé à | 200 mg |

(détail de l'excipient : lactose, talc, amidon,
stéarate de magnésium).

### RESULTATS PHARMACOLOGIQUES :

### ETUDE DE L'ACTIVITE SUR RECEPTEUR B DE L'ENDOTHELINE

On effectue une préparation membranaire à partir de cortex postérieur plus cervelet de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermediaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).

On répartit des aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits d'exemples sont données dans le tableau I ci-après, en nanomoles.

### Résultats :

**TABLEAU I**

| Produit des exemples | Récepteur B de l'endothéline CI₅₀ en nanomoles |
|---|---|
| 10 | 420 |
| 15 | 110 |
| 18 | 88 |

### ETUDE DE L'ACTIVITE SUR RECEPTEUR A DE L'ENDOTHELINE

On effectue une préparation membranaire à partir de coeur (ventricules) de rat. Le tissu est broyé au POLYTRON dans un tampon Tris 50 mM pH = 7,4.

Après 30 minutes à 25°C (B.M.) l'homogénat est centrifugé à 30000 g pendant 15 minutes (2 centrifugations avec reprise intermédiaire dans le tampon Tris pH. 7,4).

Les culots sont remis en suspension dans un tampon d'incubation (Tris 25mM, perpstatine A 5 microg/ml, aprotinine 3 microg/ml, PMSF 0,1 mM, EDTA 3mM, EGTA 1Mm pH 7,4).

On répartit des aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10-6 M (en triple). On incube à 25°C pendant 60 minutes, remet au bain-marie à 0°C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits d'exemples sont données dans le tableau II ci-après, en nanomoles.

### Résultats :

**TABLEAU II**

| Produit des exemples | Récepteur A de l'endothéline CI₅₀ en nanomoles |
|---|---|
| 10 | 167 |
| 15 | 170 |
| 18 | 230 |

## Revendications

1. Produits de formule (I) : 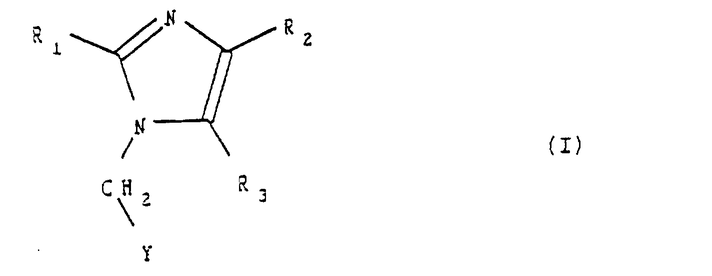 dans laquelle :
R₁ représente l'atome d'hydrogène, le radical alcoxy, 1,3-dioxolanne, dioxanne, phényle, benzoyle et phénylthio dans lesquels le radical phényle est éventuellement substitué par un radical hydroxyle ou alcoxy renfermant au plus 4 atomes de carbone,
R₂ et R₃ identiques ou différents sont choisis parmi les atomes d'halogène ; le radical carboxy libre, salifié ou estérifié par un radical alkyle renfermant au plus 4 atomes de carbone ; le radical formyle ; et les radicaux tétrazolyle, phényle, phénylthio, phénylsulfonyle, phénylsulfinyle, alkyle renfermant au plus 4 atomes de carbone, alkylthio, alkylsulfonyle et alkylsulfinyle, tous ces radicaux étant éventuellement substitués par un ou plusieurs radicaux choisis parmi les atomes d'halogène, les radicaux hydroxyle, alcoxy renfermant au plus 4 atomes de carbone, carboxy libre salifié ou estérifié et phényle lui-même éventuellement substitué par un radical hydroxyle ou alcoxy renfermant au plus 4 atomes de carbone,
et Y représente le radical 1,3-benzodioxolyl dont la partie phényle est substituée par un ou plusieurs atomes d'halogène, étant entendu que R₁ ne représente pas un radical dioxolanne
ou dioxanne lorsque R₃ représente un radical carboxy libre ou estérifié ou lorsque R₂ et R₃ représentent un atome d'halogène lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Produits de formule (I) telle que définie à la revendication 1 dans laquelle :
R₁ représente l'atome d'hydrogène, le radical 1,3-dioxolanne, phényle, benzoyle et phénylthio dans lesquels le radical phényle est éventuellement substitué par un radical hydroxyle ou alcoxy renfermant au plus 4 atomes de carbone,
R₂ représente un atome d'halogène, un radical alkylthio dans lequel le radical alkyl linéaire ou ramifié, renfermant au plus 4 atomes de carbone, est éventuellement substitué par un radical phényle lui-même éventuellement substitué par un radical alcoxy et le radical phénylthio dans lequel le radical phényle est éventuellement substitué par un radical alcoxy,
R₃ représente un radical formyle ou carboxy libre, estérifié, salifié ou amidifié, et
Y représente un radical benzodioxolyle substitué par un atome d'halogène
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

3. Les produits de formule (I) répondant aux formules suivantes :
- 1-((6-chloro-1,3-benzodioxol-5-yl) méthyl)-4-(((4-méthoxyphényl) méthyl) thio)-2-(phénylthio)-1H-imidazole-5-carboxaldéhyde
- acide 4-bromo-1-((6-chloro-1,3-benzodioxol-5-yl) méthyl)-2-phényl-1H-imidazole-5-carboxylique
- acide 1-((6-chloro-1,3-benzodioxol-5-yl) méthyl)-4-(((4-méthoxyphényl) méthyl) thio)-2-(phénylthio)-1H-imidazole-5-carboxylique
- acide 1-((6-chloro-1,3-benzodioxol-5-yl) méthyl)-4-(4-méthoxyphényl) thio)-2-(phénylthio)-1H-imidazole-5-carboxylique
- acide 1-((6-chloro-1,3-benzodioxol-5-yl) méthyl)-2,4-bis(4-(méthoxyphényl) thio)-1H-imidazole-5-carboxylique
- acide 1-((6-chloro 1,3-benzodioxol-5-yl) méthyl) 4-((3,4-diméthoxyphényl) thio) 2-(1,3-dioxolan-2-yl) 1H-imidazol 5-carboxylique, ainsi que leurs sels d'addition avec les acides minéraux et organiques ou avec les bases minérales et organiques.

4. Procédé de préparation des produits de formule (I), telle que définie à la revendication 1, **caractérisé en ce que** :
soit l'on soumet un composé de formule (II) : 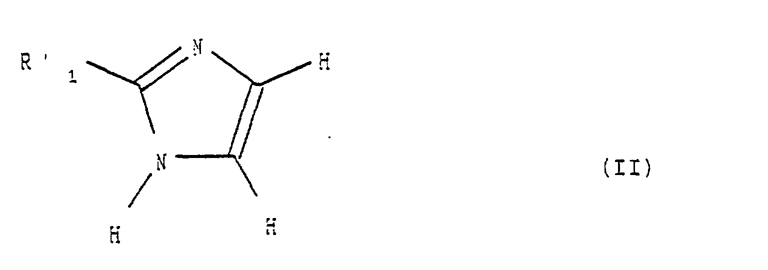
dans laquelle R'₁ a la signification indiquée à la revendication 1 pour R₁, dans laquelle les éventuelles fonctions réactives sont éventuellement protégées par des groupements protecteurs,
à une réaction avec un composé de formule (III) : 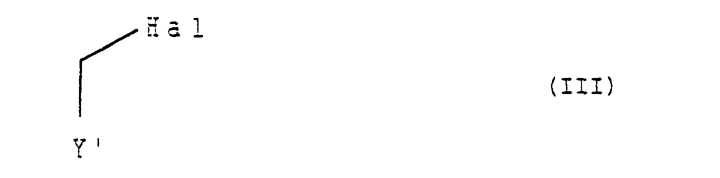 dans laquelle Hal représente un atome d'halogène, et
Y' a la signification indiquée à la revendication 1 pour Y,
dans laquelle les éventuelles fonctions réactives sont éventuellement protégées par des groupements protecteurs,
pour obtenir le produit de formule (IV₁) : 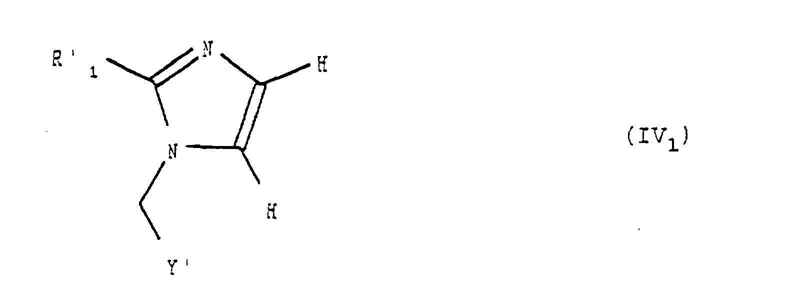 dans laquelle R'₁ et Y' ont les significations indiquées ci-dessus, produit de formule (IV₁) que l'on peut soumettre à une réaction d'halogénation, pour obtenir le produit de formule (IV₂) : 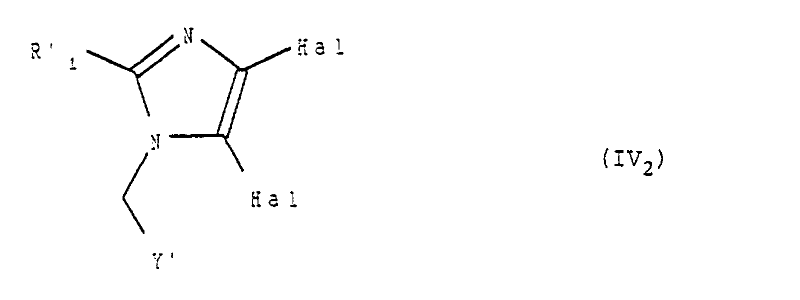 dans laquelle R'₁, Hal et Y' ont les significations indiquées ci-dessus,
soit l'on soumet un composé de formule (VIII) : 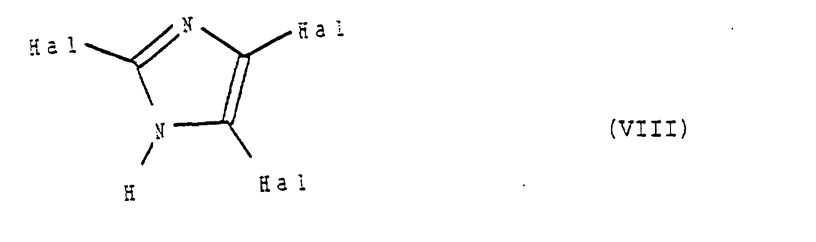 dans laquelle Hal a la signification indiquée ci-dessus,
soit à une réaction avec le composé de formule (III) telle que définie ci-dessus, soit à l'action d'un groupement protecteur P, pour obtenir un produit de formule (IX) : 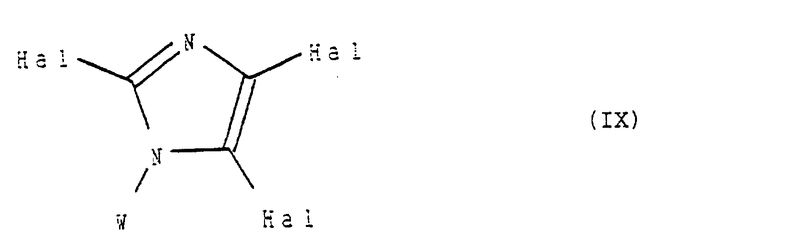 dans laquelle Hal a la signification indiquée ci-dessus et W représente soit -CH₂-Y' avec Y' tel que défini ci-dessus,
soit P qui représente un groupement protecteur de l'atome d'azote, produit de formule (IX) que l'on soumet à une réaction d'échange halogène-métal puis à une réaction avec un électrophile de formule (X), (X'), (XI), (XII) ou (XII') :
L₁-CHO (X)
L₁-CO-Cl (X')
(-S-L₁)₂ (XI)
L₁SO₂-S-L'₁ (XII)
L₁SO₂-Cl (XII')
dans lesquelles L₁ et L'₁ identiques ou différents, représentent un radical alkyle, alkényle ou aryle, tels que définis ci-dessus, pour obtenir un produit de formule (XIII) : 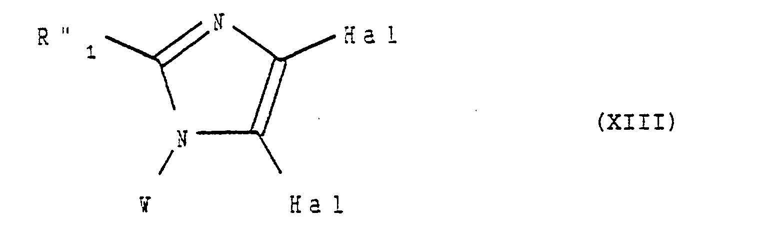 dans laquelle Hal et W ont les significations indiquées ci-dessus, R"₁ représente un radical aryl-, alkyl-carbonyle,
aryl- ou alkyl- hydroxyméthyl, ou arylthio dans lesquels les radicaux aryle et alkyle ont les significations indiquées ci-dessus et dans lesquels les éventuelles fonctions réactives sont éventuellement protégées par des groupements protecteurs,
produits de formules (IV₂) et (XIII) que l'on peut soumettre à une réaction d'échange halogène-métal sur l'un des atomes d'halogène puis à une réaction avec CO₂ ou DMF ou un composé électrophile de formule (Vₐ), (V_{b}), (VIₐ), (VI_{b}) ou (VI_{c}) :
(-S-Z₁)₂ (Va)
ou
MeSO₂SZ₁ (V_{b})
ou 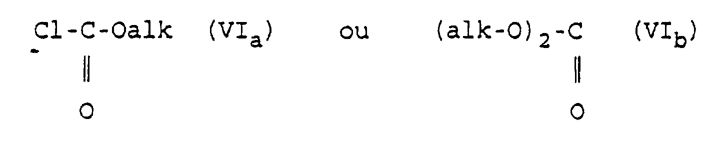 ou 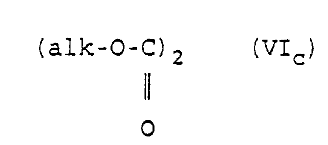 dans lesquelles Z₁ représente un radical alkyle, alkényle ou aryle, éventuellement substitués, dans lesquelles les éventuelles fonctions réactives sont éventuellement protégées par des groupements protecteurs, et alk représente un radical alkyle renfermant au plus 4 atomes de carbone,
pour obtenir respectivement le composé de formule (I₁) : 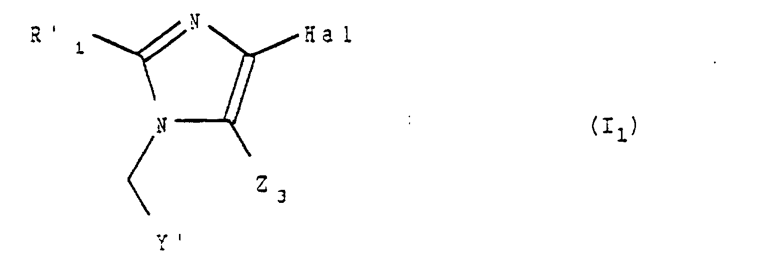 ou le composé de formule (XIV) : 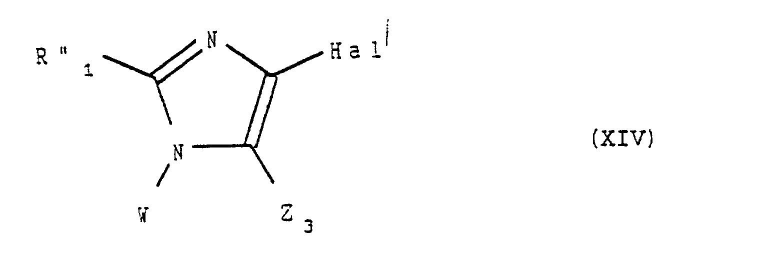 dans lesquelles R'₁, R"₁, Hal, Y' et W ont les significations indiquées ci-dessus et Z₃ représente le radical carboxy, formyle, S-Z₁ tel que défini ci-dessus, ou le radical K-O-alk dans lequel K représente le radical 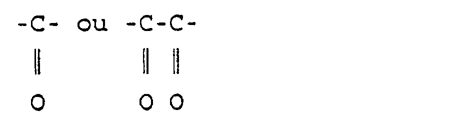 et alk a la signification indiquée ci-dessus,
composés de formule (I₁) ou (XIV) que lorsque Z₃ représente un radical formyle ou carboxy estérifié, l'on peut soumettre à une réaction avec un composé de formule (VII) :
Z₂-S-M (VII)
dans laquelle S représente un atome de soufre, M représente un métal tel que sodium, potassium ou cuivre et Z₂ représente un radical alkyle, alkényle ou aryle, éventuellement substitué, dans lesquelles les éventuelles fonctions réactives sont éventuellement protégées par des groupements protecteurs pour obtenir respectivement le composé de formule (I₂) : 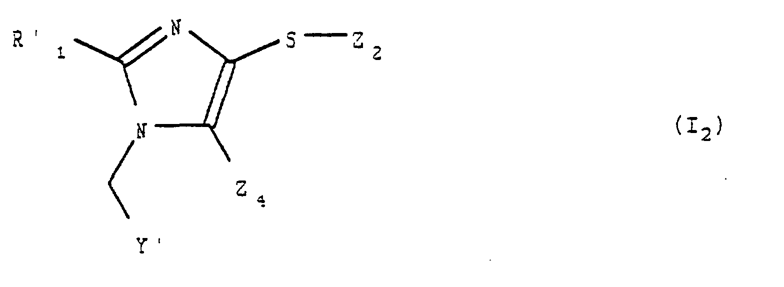 ou le composé de formule (XV) : 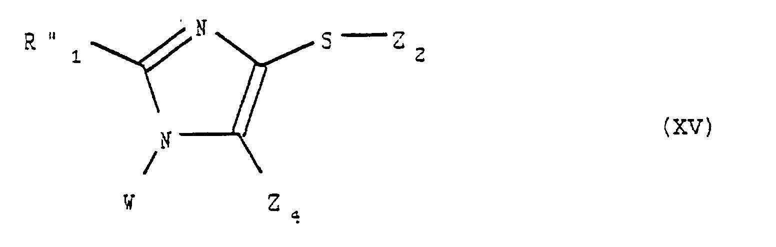 dans lesquelles R'₁, R"₁, Y', Z₂ et W ont les significations indiquées ci-dessus, et Z₄ représente le radical formyle ou carboxy estérifié, produits de formules (I₂) et (XV) que lorsque Z₄ représente le radical formyle, l'on peut soumettre à une réaction d'oxydation ou de réduction pour obtenir respectivement un produit de formule (I₃) : 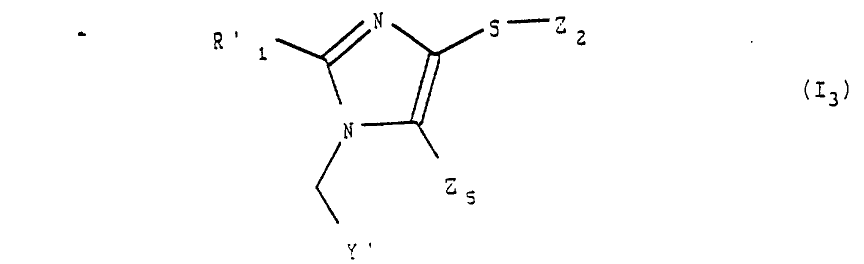 ou un produit de formule (I₄) : 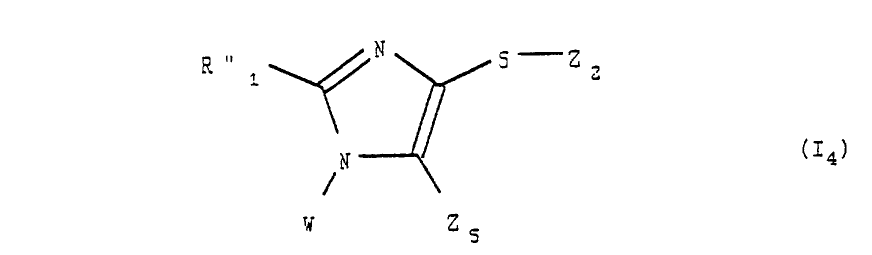 dans lesquelles R'₁, R"₁, Z₂, Y' et W ont les significations indiquées ci-dessus, et Z₅ représente le radical CH₂OH ou le radical carboxy libre ou estérifié par un radical alkyle linéaire ou ramifié renfermant au plus 6 atomes de carbone, produits de formule (XV) ou (I₄) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (I₅) : 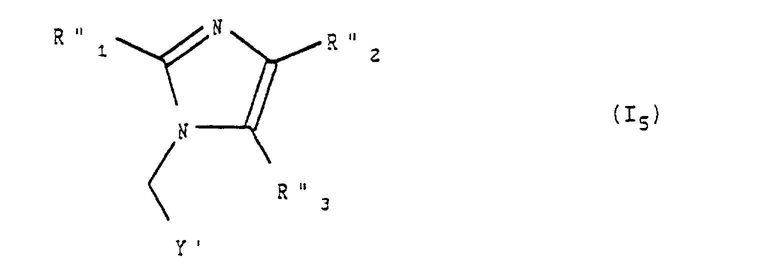 dans laquelle R"₁ et Y' ont les significations indiquées ci-dessus, et R"₂ et R"₃ représentent indiféremment l'un Z₄ ou Z₅ tels que définis ci-dessus et l'autre S-Z₂, tel que défini ci-dessus,
soit l'on soumet un composé de formule (XVI) : 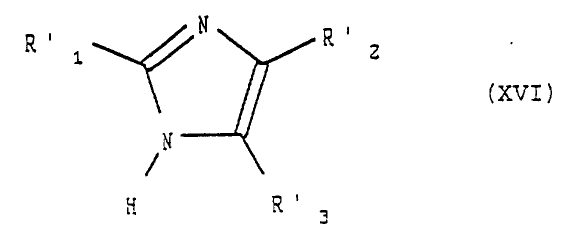 dans laquelle R'₁ a la signification indiquée ci-dessus et R'₂ et R'₃ ont les significations indiquées à la revendication 1 respectivement pour R₂ et R₃ dans lesquelles les éventuelles fonctions réactives sont éventuellement protégées par des groupements protecteurs, à une réaction avec le composé de formule (III) telle que définie ci-dessus, pour obtenir un produit de formule (I') : 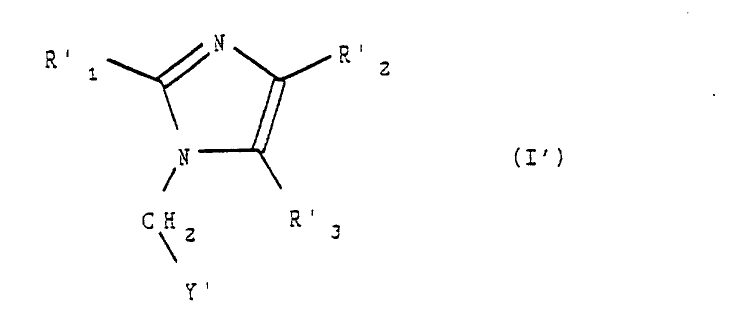 dans laquelle R'₁, R'₂, R'₃ et Y' ont les définitions indiquées ci-dessus,
produits de formules (I₁), (I₂), (I₃), (I₄), (XIV), (XV), (I₅) et (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction de transformation de fonction ester en fonction acyle,
d) une réaction de transformation de la fonction cyano en fonction acide,
e) une réaction de transformation de fonction acide en fonction amide, puis éventuellement en fonction thioamide,
f) une réaction de réduction de la fonction carboxy en fonction alcool,
g) une réaction de transformation de fonction alcoxy en fonction hydroxyle, ou encore de fonction hydroxyle en fonction alcoxy,
h) une réaction d'oxydation de fonction alcool en fonction aldéhyde, acide ou cétone,
i) une réaction de transformation du radical formyle en radical carbamoyle,
j) une réaction de transformation du radical carbamoyle en radical nitrile,
k) une réaction de transformation de radical nitrile en tétrazolyle,
l) une réaction d'oxydation de groupement alkylthio ou arylthio en sulfoxyde ou sulfone correspondant,
m) une réaction de transformation de fonction sulfure, sulfoxyde ou sulfone en fonction sulfoximine correspondante,
n) une réaction de transformation de fonction oxo en fonction thioxo,
o) une réaction de transformation du radical 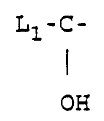 en radical 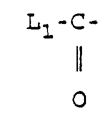 puis si nécessaire de nouveau en radical 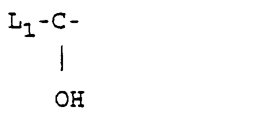 avec L₁ tel que défini ci-dessus,
p) une réaction de transformation de fonction acide en fonction 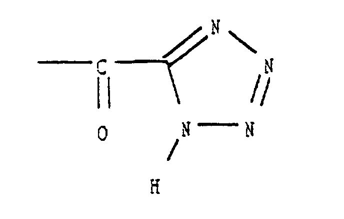
q) une réaction de transformation de la fonction β-cétosulfoxyde en fonction α-céto thio ester,
r) une-réaction de transformation d'un carbamate en urée et notamment d'un sulfonylcarbamate en sulfonylurée,
s) une réaction d'élimination des groupements protecteurs que peuvent porter les fonctions réactives protégées,
t) une réaction de salification par un acide minéral ou organique ou par une base pour obtenir le sel correspondant,
u) une réaction de dédoublement des formes racémiques en produits dédoublés,
lesdits produits de formule (I) ainsi obtenus étant sous toutes les formes isomères possibles racémiques, énantiomères et diastéréoisomères.

5. A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

6. A titre de médicaments, les produits de formule (I) tels que définis aux revendications 2 et 3, ainsi que les sels d'addition avec les acides minéraux et organiques ou avec les bases minérales et organiques pharmaceutiquement acceptables desdits produits de formule (I).

7. Les compositions pharmaceutiques contenant à titre de principe actif, l'un au moins des médicaments tels que définis à l'une quelconque des revendications 5 ou 6.

8. Utilisation des produits de formule (I) telle que définie à la revendication 1, pour la préparation de compositions pharmaceutiques destinées au traitement d'affections résultant d'une stimulation anormale des récepteurs de l'endothéline.

9. Utilisation des produits de formule (I) telle que définie à la revendication 1, pour la préparation de compositions pharmaceutiques destinées au traitement de l'hypertension induite par l'endothéline, de tous les spasmes vasculaires et du traitement des post-hémorragies cérébrales et des insuffisances rénales, de l'infarctus du myocarde et à la prévention des resténoses post-angioplastie.

## Claims

1. Products of formula (I): 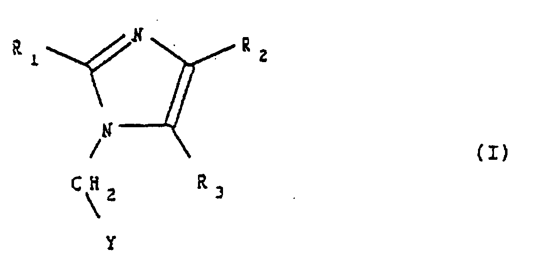 in which:
R₁ represents the hydrogen atom; the alkoxy, 1,3-dioxolanyl, dioxanyl, phenyl, benzoyl and phenylthio radical in which the phenyl radical is optionally substituted by a hydroxyl radical or by an alkoxy radical including at most 4 carbon atoms,
R₂ and R₃, which are identical or different, are chosen from halogen atoms; the carboxyl radical, which is free, salified or esterified by an alkyl radical including at most 4 carbon atoms; the formyl radical; and tetrazolyl, phenyl, phenylthio, phenylsulphonyl, phenylsulphinyl, alkyl, including at most 4 carbon atoms, alkylthio, alkylsulphonyl and alkylsulphinyl radicals, all these radicals optionally being substituted by one or more radicals chosen from halogen atoms, the hydroxyl radical, the alkoxy radical including at most 4 carbon atoms, the free, salified or esterified carboxyl radical and the phenyl radical, itself optionally substituted by a hydroxyl radical or by an alkoxy radical including at most 4 carbon atoms, and Y represents the 1,3-benzodioxolyl radical, the phenyl part of which is substituted by one or more halogen atoms, it being understood that R₁ does.not represent a dioxolanyl or dioxanyl radical when R₃ represents a free or esterified carboxyl radical or when R₂ and R₃ represent a halogen atom, the said products of formula (I) being in all the isomeric forms possible, racemic, enantiomeric and diastereoisomeric, and the addition salts with inorganic and organic acids or with inorganic and organic bases of the said products of formula (I).

2. Products of formula (I) as defined in Claim 1, in which:
R₁ represents the hydrogen atom; the 1,3-dioxolanyl, phenyl, benzoyl and phenylthio radical in which the phenyl radical is optionally substituted by a hydroxyl radical or by an alkoxy radical including at most 4 carbon atoms,
R₂ represents a halogen atom, an alkylthio radical, in which the linear or branched alkyl radical, including at most 4 carbon atoms, is optionally substituted by a phenyl radical itself optionally substituted by an alkoxy radical, and the phenylthio radical, in which the phenyl radical is optionally substituted by an alkoxy radical,
R₃ represents a formyl radical or a free, esterified, salified or amidated carboxyl radical, and
Y represents a benzodioxolyl radical substituted by a halogen atom,
the said products of formula (I) being in all the isomeric forms possible, racemic, enantiomeric and diastereoisomeric, and the addition salts with inorganic and organic acids or with inorganic and organic bases of the said products of formula (I).

3. The products of formula (I) corresponding to the following formulae:
- 1-((6-chloro-1,3-benzodioxol-5-yl)methyl)-4-(((4-methoxyphenyl)methyl)thio)-2-(phenylthio)-1H-imidazole-5-carboxaldehyde
- 4-bromo-1-((6-chloro-1,3-benzodioxol-5-yl)methyl)-2-phenyl-1H-imidazole-5-carboxylic acid
- 1-((6-chloro-1,3-benzodioxol-5-yl)methyl)-4-(((4-methoxyphenyl)methyl)thio)-2-(phenylthio)-lH-imidazole-5-carboxylic acid
- 1-((6-chloro-1,3-benzodioxol-5-yl)methyl)-4-((4-methoxyphenyl)thio)-2-(phenylthio)-1H-imidazole-5-carboxylic acid
- 1-((6-chloro-1,3-benzodioxol-5-yl)methyl)-2,4-bis((4-methoxyphenyl)thio)-1H-imidazole-5-carboxylic acid
- 1-((6-chloro-1,3-benzodioxol-5-yl)methyl)-4-((3,4-dimethoxyphenyl)thio)-2-(1,3-dioxolan-2-yl)-1H-imidazole-5-carboxylic acid,
and their addition salts with inorganic and organic acids or with inorganic and organic bases.

4. Process for the preparation of the products of formula (I) as defined in Claim 1, **characterized in that**:
either a compound of formula (II): 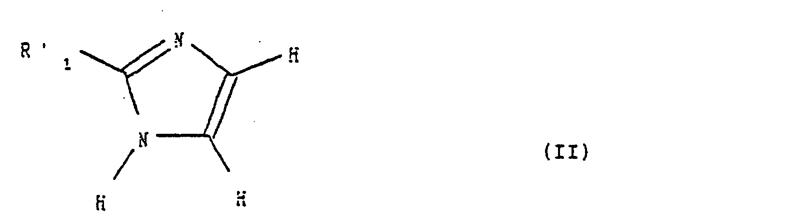 in which R'₁ has the meaning indicated in Claim 1 for R₁, in which the possible reactive functional groups are optionally protected by protective groups,
is reacted with a compound of formula (III): 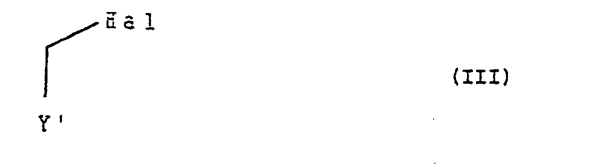 in which Hal represents a halogen atom and Y' has the meaning indicated in Claim 1 for Y, in which the
possible reactive functional groups are optionally protected by protective groups,
to produce the product of formula (IV₁): 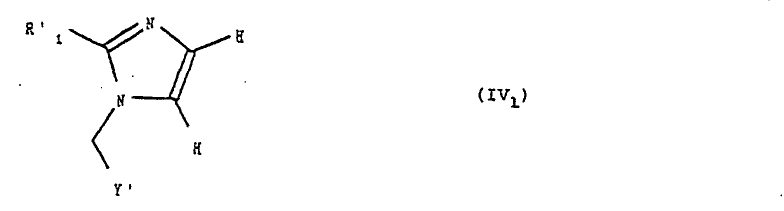 in which R'₁ and Y' have the meanings indicated above, which product of formula (IV₁) can be subjected to a halogenation reaction, to produce the product of formula (IV₂) : 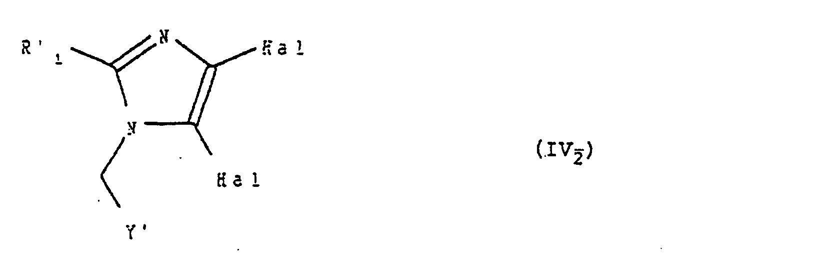 in which R'₁, Hal and Y' have the meanings indicated above,
or a compound of formula (VIII): 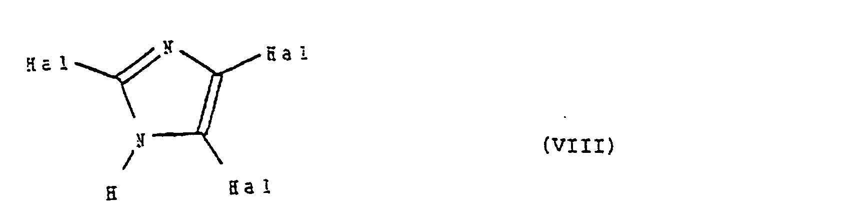 in which Hal has the meaning indicated above,
is either reacted with the compound of formula (III) as defined above or is subjected to the action of a protective group P, to produce a product of formula (IX) : 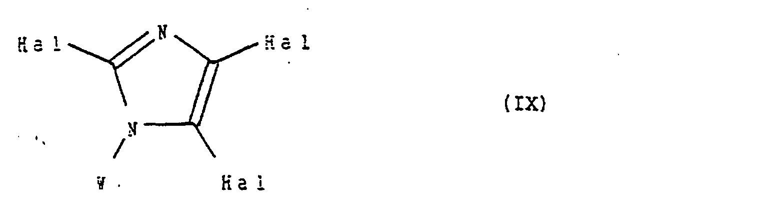 in which Hal has the meaning indicated above and W represents either -CH₂-Y', with Y' as defined above, or P, which represents a protective group for the nitrogen atom, which product of formula (IX) is subjected to a halogen-metal exchange reaction and is then reacted with an electrophile of formula (X), (X'), (XI), (XII) or (XII') :
L₁-CHO (X)
L₂-CO-Cl (X')
(-S-L₁)₂ (XI)
L₁SO₂-S-L'₁ (XII)
L₁SO₂-Cl (XII')
in which L₁ and L'₁, which are identical or different, represent an alkyl, alkenyl or aryl radical as defined above, to produce a product of formula (XIII): 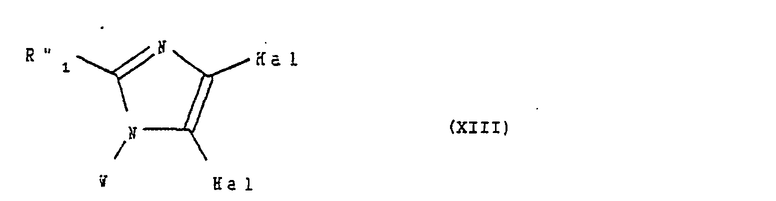 in which Hal and W have the meanings indicated above and R"₁ represents an arylcarbonyl, alkylcarbonyl, arylhydroxymethyl, alkylhydroxymethyl or arylthio radical in which the aryl and alkyl radicals have. the meanings indicated above and in which the possible reactive functional groups are optionally protected by protective groups,
which products of formulae (IV₂) and (XIII) can be subjected to a halogen-metal exchange reaction on one of the halogen atoms and can then be reacted with CO₂ or DMF or an electrophilic compound of formula (Vₐ), (V_{b}), (VIₐ), (VI_{b}) or (VI_{c}) :
(-S-Z₁)₂ (Vₐ) or MeSO₂SZ₁ (V_{b}) or 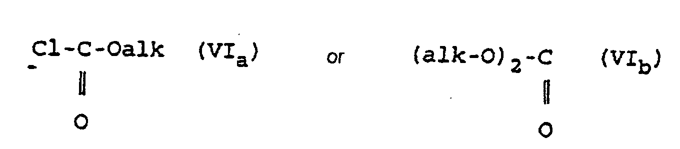
or 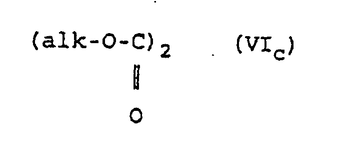 in which Z₁ represents an optionally substituted alkyl, alkenyl or aryl radical, in which formulae the possible reactive functional groups are optionally protected by protective groups, and alk represents an alkyl radical including at most 4 carbon atoms,
to respectively produce the compound of formula (I₁): 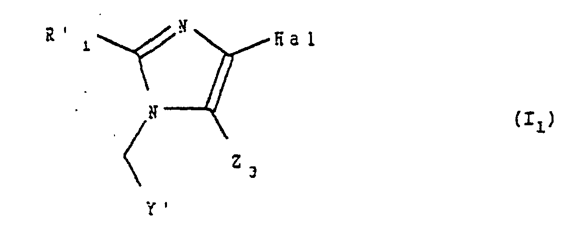 or the compound of formula (XIV): 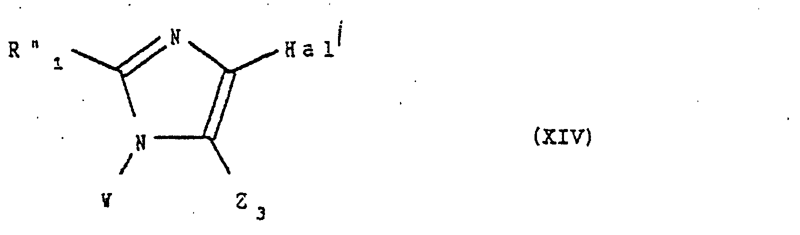 in which R'₁, R"₁, Hal, Y' and W have the meanings indicated above and Z₃ represents the carboxyl radical, the formyl radical, the S-Z₁ radical as defined above or the K-O-alk radical in which K represents the 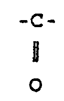 or 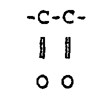 radical and alk has the meaning indicated above, which compounds of formula (I₁) or (XIV), when Z₃ represents a formyl or esterified carboxyl radical, can be reacted with a compound of formula (VII) :
Z₂-S-M (VII)
in which S represents a sulphur atom, M represents a metal, such as sodium, potassium or copper, and Z₂ represents an optionally substituted alkyl, alkenyl or aryl radical, in which formulae the possible reactive functional groups are optionally protected by protective groups, to respectively produce the compound of formula (I₂) : 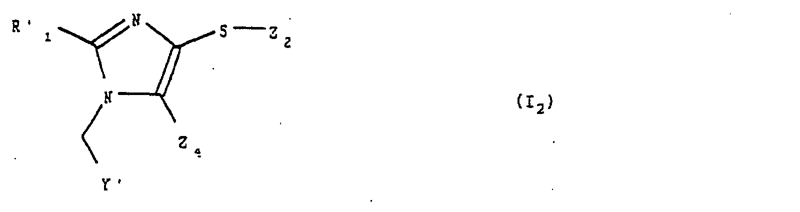 or the compound of formula (XV): 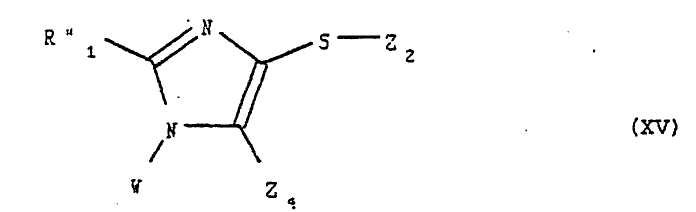 in which R'₁, R"₁, Y', Z₂ and W have the meanings indicated above and Z₄ represents the formyl or esterified carboxyl radical, which products of formulae (I₂) and (XV), when Z₄ represents the formyl radical, can be oxidized or reduced, to respectively produce a product of formula (I₃) : 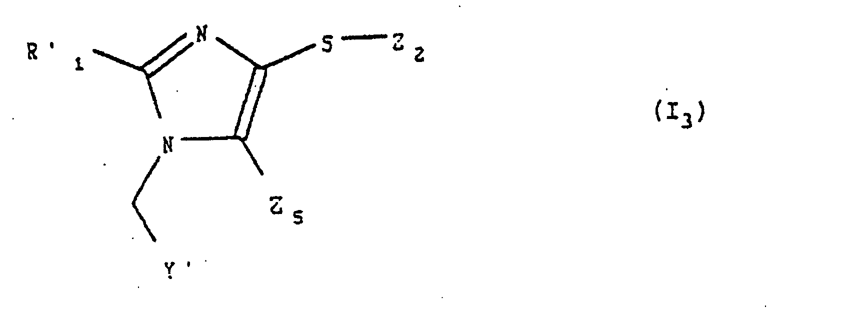 or a product of formula (I₄): 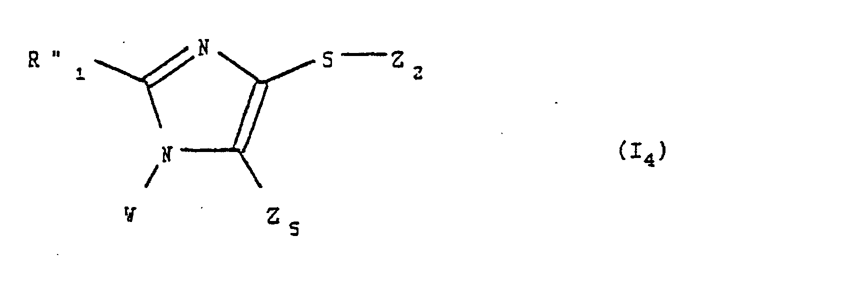 in which R'₁, R"₁, Z₂, Y' and W have the meanings indicated above and Z₅ represents the CH₂OH radical or the carboxyl radical, free or esterified by a linear or branched alkyl radical including at most 6 carbon atoms, which products of formula (XV) or (I₄), in the case where W represents P as defined above and after release of the amine functional group blocked by P as defined above, are reacted with the compound of formula (III) as defined above, to produce a product of formula (I₅): 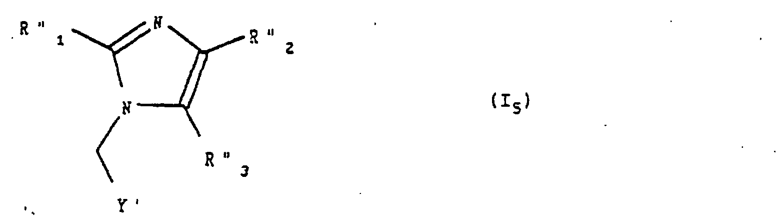 in which R"₁ and Y' have the meanings indicated above and R"₂ and R"₃ represent, without distinction, one Z₄ or Z₅ as defined above and the other S-Z₂ as defined above,
or a compound of formula (XVI) : 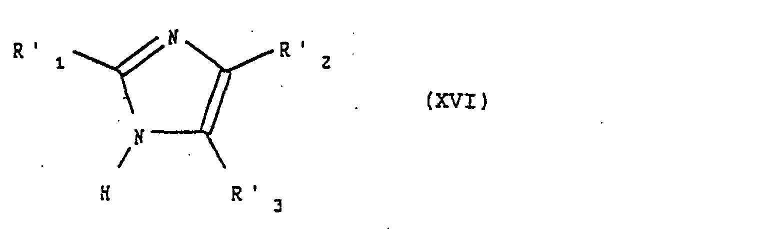 in which R'₁ has the meaning indicated above and R'₂ and R'₃ have the meanings indicated in Claim 1 respectively for R₂ and R₃, in which formulae the possible reactive functional groups are optionally protected by protective groups, is reacted with the compound of formula (III) as defined above, to produce a product of formula (I'): 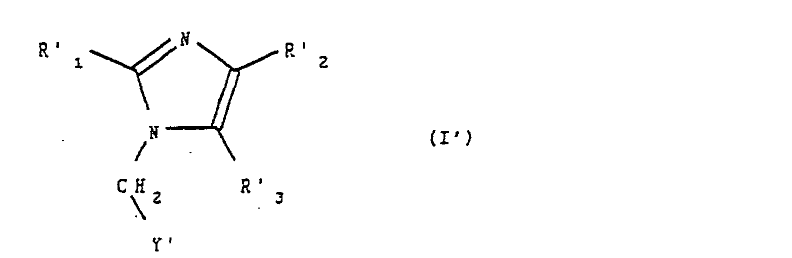 in which R'₁, R'₂, R'₃ and Y' have the definitions indicated above,
which products of formulae (I₁), (I₂), (I₃), (I₄), (XIV), (XV), (I₅) and (I') can be products of formula (I) and, to produce products or other products of formula (I), can be subjected, if desired and if necessary, to one or more of the following conversion reactions, in. any order:
a) a reaction for esterification of an acid functional group,
b) a reaction for saponification of an ester functional group to an acid functional group,
c) a reaction for conversion of an ester functional group to an acyl functional group,
d) a reaction for conversion of the cyano functional group to an acid functional group,
e) a reaction for conversion of an acid functional group to an amide functional group and then optionally to a thioamide functional group,
f) a reaction for reduction of the carboxyl functional group to an alcohol functional group,
g) a reaction for conversion of an alkoxy functional group to a hydroxyl functional group or alternatively of a hydroxyl functional group to an alkoxy functional group,
h) a reaction for oxidation of an alcohol functional group to an aldehyde, acid or ketone functional group,
i) a reaction for conversion of the formyl radical to a carbamoyl radical,
j) a reaction for conversion of the carbamoyl radical to a nitrile radical,
k) a reaction for conversion of a nitrile radical to tetrazolyl,
l) a reaction for oxidation of an alkylthio or arylthio group to the corresponding sulphoxide or sulphone,
m) a reaction for conversion of a sulphide, sulphoxide or sulphone functional group to the corresponding sulphoximine functional group,
n) a reaction for conversion of an oxo functional group to a thioxo functional group,
o) a reaction for conversion of the 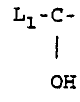 radical to an 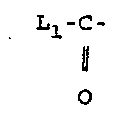 radical and then, if necessary, again to an 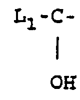 radical with L₁ as defined above,
p) a reaction for conversion of an acid functional group to a functional group 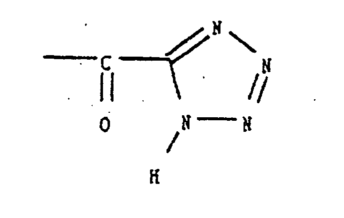
q) a reaction for conversion of the β-ketosulphoxide functional group to an α-ketothioester functional group,
r) a reaction for conversion of a carbamate to a urea and in particular of a sulphonylcarbamate to a sulphonylurea,
s) a reaction for removal of the protective groups which the protected reactive functional groups can carry,
t) a reaction for salification by an inorganic or organic acid or by a base, to produce the corresponding salt,
u) a reaction for resolution of the racemic forms to give resolved products,
the said products of formula (I) thus obtained being in all the isomeric forms possible, racemic, enantiomeric and diastereoisomeric.

5. As medicaments, the products as defined by the formula (I) of Claim 1, the said products of formula (I) being in all the isomeric forms possible, racemic, enantiomeric and diastereoisomeric, and the addition salts with pharmaceutically acceptable inorganic and organic acids or with pharmaceutically acceptable inorganic and organic bases of the said products of formula (I).

6. As medicaments, the products of formula (I) as defined in Claims 2 and 3, and the addition salts with pharmaceutically acceptable inorganic and organic acids or with pharmaceutically acceptable inorganic and organic bases of the said products of formula (I).

7. The pharmaceutical compositions comprising, as active principle, at least one of the medicaments as defined in either one of Claims 5 and 6.

8. Use of the products of formula (I) as defined in Claim 1 in the preparation of pharmaceutical compositions intended for the treatment of conditions resulting from abnormal stimulation of endothelin receptors.

9. Use of the products of formula (I) as defined in Claim 1 in the preparation of pharmaceutical compositions intended for the treatment of endothelininduced hypertension, of all vascular spasms, of cerebral post-haemorrhage, of renal insufficiency and of myocardial infarction and for the prevention of post-angioplastic restenosis.

## Patentansprüche

1. Produkte der Formel (I) 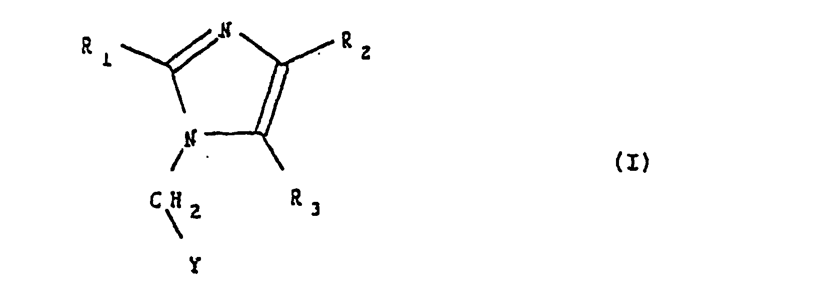 in der
R₁ das Wasserstoffatom, den Rest Alkoxy, 1,3-Dioxolan, Dioxan, Phenyl, Benzoyl und Phenylthio darstellt, worin der Rest Phenyl gegebenenfalls substituiert ist durch einen Rest Hydroxyl oder Alkoxy mit höchstens 4 Kohlenstoffatomen,
R₂ und R₃, gleich oder verschieden, ausgewählt werden unter den Halogenatomen; dem Rest Carboxy, frei, in Salz überführt oder verestert durch einen Rest Alkyl mit höchstens 4 Kohlenstoffatomen; dem Rest Formyl; und den Resten Tetrazolyl, Phenyl, Phenylthio, Phenylsulfonyl, Phenylsulfinyl, Alkyl mit höchstens 4 Kohlenstoffatomen, Alkylthio, Alkylsulfonyl und Alkylsulfinyl,
wobei alle diese Reste gegebenenfalls substituiert sind durch einen oder mehrere Reste, ausgewählt unter den Halogenatomen, den Resten Hydroxyl, Alkoxy mit höchstens 4 Kohlenstoffatomen, Carboxy, frei, in Salz überführt oder verestert und Phenyl, das gegebenenfalls selbst substituiert ist durch einen Rest Hydroxyl oder Alkoxy mit höchstens 4 Kohlenstoffatomen, und
Y den Rest 1,3-Benzodioxolyl bedeutet, dessen Teil Phenyl substituiert ist durch ein oder mehrere Halogenatome, mit der Maßgabe, daß R₁ keinen Rest Dioxolan oder Dioxan darstellt, wenn R₃ ein
Rest Carboxy ist, frei oder verestert, oder wenn R₂ und R₃ ein Halogenatom bedeuten,
wobei die genannten Produkte der Formel (I) in allen möglichen isomeren racemischen Formen, Enantiomeren und Diastereoisomeren vorliegen können, sowie die Additionssalze der genannten Produkte der Formel (I) mit Mineralsäuren und organischen Säuren oder mit Mineralbasen und organischen Basen.

2. Produkte der Formel (I) wie in Anspruch 1 definiert, in der in der
R₁ das Wasserstoffatom, den Rest 1,3-Dioxolan, Phenyl, Benzoyl und Phenylthio darstellt, worin der Rest Phenyl gegebenenfalls substituiert ist durch einen Rest Hydroxyl oder Alkoxy mit höchstens 4 Kohlenstoffatomen,
R₂ ein Halogenatom, einen Rest Alkylthio, worin der Rest Alkyl linear oder verzweigt ist und höchstens 4 Kohlenstoffatome umfaßt, gegebenenfalls substituiert durch einen Rest Phenyl, der gegebenenfalls selbst substituiert ist durch einen Rest Alkoxy, und den Rest Phenylthio darstellt, worin der Rest Phenyl gegebenenfalls substituiert ist durch einen Rest Alkoxy,
R₃ einen Rest Formyl oder Carboxy, frei, verestert, in Salz überführt oder amidiert, bedeutet und
Y einen Rest Benzodioxolyl darstellt, substituiert durch ein Halogenatom,
wobei die genannten Produkte der Formel (I) in allen möglichen isomeren racemischen Formen, Enantiomeren und Diastereoisomeren vorliegen können, sowie die Additionssalze der genannten Produkte der Formel (I) mit Mineralsäuren und organischen Säuren oder mit Mineralbasen und organischen Basen.

3. Produkte der Formel (I), die den folgenden Formeln entsprechen:
- 1-[(6-Chlor-1,3-benzodioxol-5-yl)-methyl)-4-{[(4-methoxyphenyl)-methyl]-thio}-2-(phenylthio)-1H-imidazol-5-carboxaldehyd
- 4-Brom-1-[(6-chlor-1,3-benzodioxol-5-yl)-methyl]-2-phenyl-1Himidazol-5-carbonsäure
- 1-[(6-Chlor-1,3-benzodioxol-5-yl)-methyl]-4-{[(4-methoxyphenyl)-methyl]-thio}-2-(phenylthio)-1H-imidazol-5-carbonsäure
- 1-[(6-Chlor-1,3-benzodioxol-5-yl)-methyl]-4-[(4-methoxyphenyl)-thio]-2-(phenylthio)-1H-imidazol-5-carbonsäure
- 1-[(6-Chlor-1,3-benzodioxol-5-yl)-methyl]-2,4-bis-[(4-methoxyphenyl)-thio]-1H-imidazol-5-carbonsäure
- 1-[(6-Chlor-1,3-benzodioxol-5-yl)-methyl]-4-[(3,4-dimethoxyphenyl)-thio]-2-(1,3-dioxolan-2-yl)-1H-imidazol-5-carbonsäure
sowie ihre Additionssalze mit Mineralsäuren und organischen Säuren oder mit Mineralbasen und organischen Basen.

4. Verfahren zur Herstellung der Produkte der Formel (I) wie in Anspruch 1 definiert, **dadurch gekennzeichnet, daß** man
entweder eine Verbindung der Formel (II) 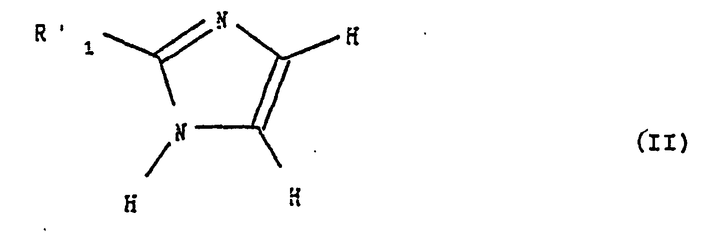 in der R'₁ die in Anspruch 1 für R₁ angegebene Bedeutung besitzt und worin die eventuellen reaktiven Funktionen gegebenenfalls durch Schutzgruppen geschützt sind, einer Reaktion mit einer Verbindung der Formel (III) 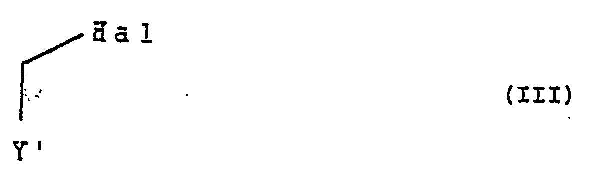 unterzieht, in der Hal ein Halogenatom ist und Y' die in Anspruch 1 für Y angegebene Bedeutung besitzt, und worin die eventuellen reaktiven Funktionen gegebenenfalls durch Schutzgruppen geschützt sind, um das Produkt der Formel (IV₁) 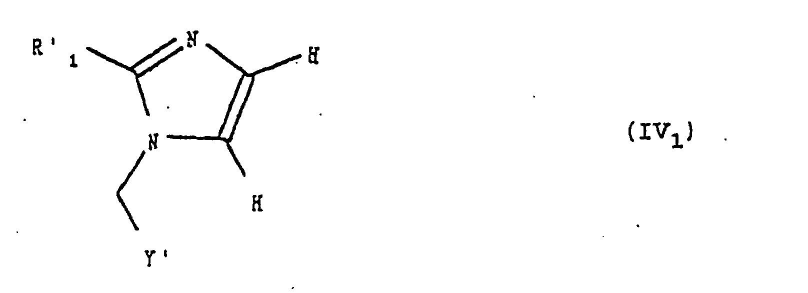 zu erhalten, in der R'₁ und Y' die oben angegebenen Bedeutungen besitzen, das man einer Reaktion der Halogenierung unterziehen kann, um das Produkt der Formel (IV₂) 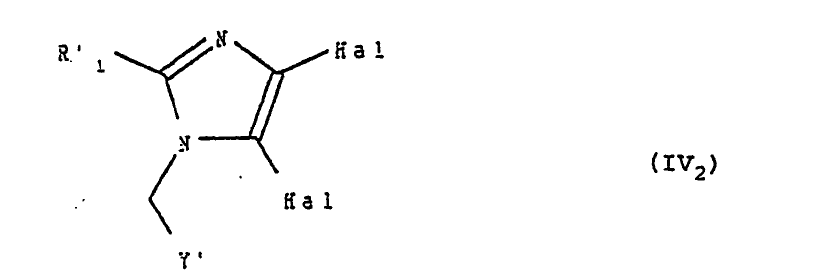 zu erhalten, in der R'₁, Hal und Y' die oben angegebenen Bedeutungen besitzen,
oder eine Verbindung der Formel (VIII) 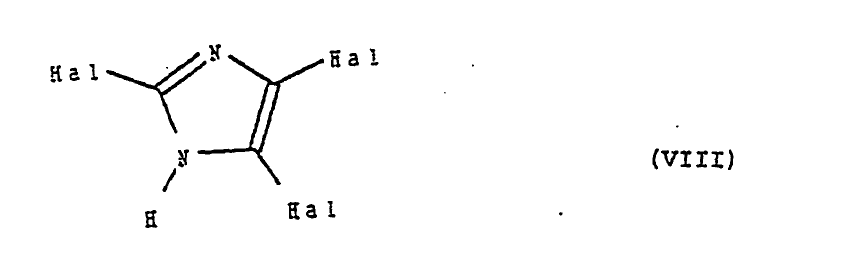 in der Hal die oben angegebene Bedeutung besitzt, entweder einer Reaktion mit einer Verbindung der Formel (III), wie oben definiert, oder der Einwirkung einer Schutzgruppe P unterzieht, um ein Produkt der Formel (IX) 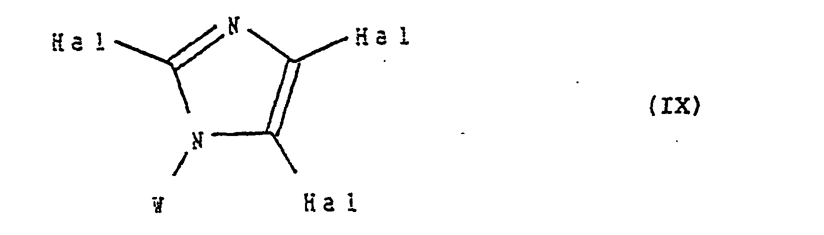 zu erhalten, in der Hal die oben angegebene Bedeutung besitzt und W entweder -CH₂-Y' mit Y' wie oben definiert oder P darstellt, das eine Schutzgruppe für das Stickstoffatom bedeutet, und man das Produkt der Formel (IX) einer Reaktion zum Austausch Halogen-Metall und anschließend einer Reaktion mit einem Elektrophil der Formeln (X), (X'), (XI), (XII) oder (XII')
L₁-CHO (X)
L₁-CO-Cl (X')
(-S-L₁)₂ (XI)
L₁SO₂-S-L'₁ (XII)
L₁SO₂-Cl (XII')
unterzieht, in denen L₁ und L'₁, gleich oder verschieden, einen Rest Alkyl, Alkenyl oder Aryl darstellen, wie oben definiert, um ein Produkt der Formel (XIII) 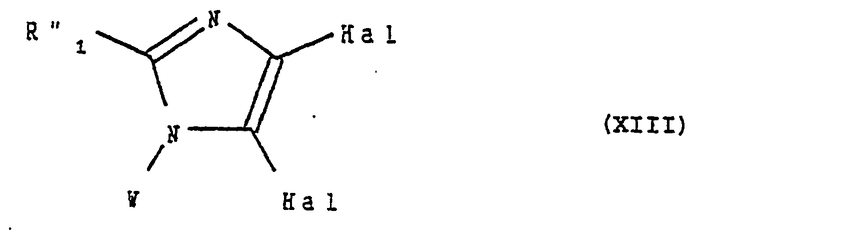 zu erhalten, in der Hal und W die oben angegebenen Bedeutungen besitzen, R"₁ einen Rest Aryl-, Alkyl-carbonyl, Aryl- oder Alkylhydroxymethyl oder Arylthio darstellt, in denen die Reste Aryl und Alkyl die oben angegebenen Bedeutungen besitzen und in denen die eventuellen reaktiven Funktionen gegebenenfalls durch Schutzgruppen geschützt sind,
und man die Produkte der Formeln (IV₂) und (XIII) gegebenenfalls einer Reaktion zum Austausch Halogen-Metall an einem der Halogenatome und anschließend einer Reaktion mit CO₂ oder DMF oder einer elektrophilen Verbindung der Formeln (Vₐ), (V_{b}) , (VIₐ), (VI_{b}) oder (VI_{c})
(-S-Z₁)₂ (Vₐ) oder MeSO₂SZ₁ (V_{b}) oder 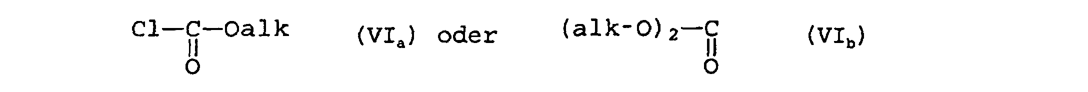
oder 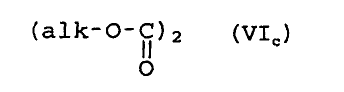 unterzieht, in denen Z₁ einen Rest Alkyl, Alkenyl oder Aryl darstellt, gegebenenfalls substituiert, und in denen die eventuellen reaktiven Funktionen gegebenenfalls durch Schutzgruppen geschützt sind, und alk einen Rest Alkyl mit höchstens 4 Kohlenstoffatomen bedeutet,
um jeweils die Verbindung der Formel (I₁) 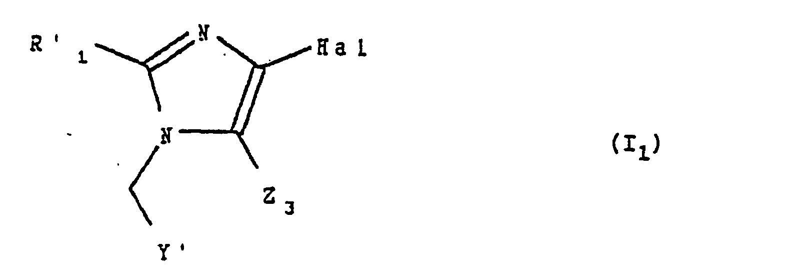 oder die Verbindung der Formel (XIV) 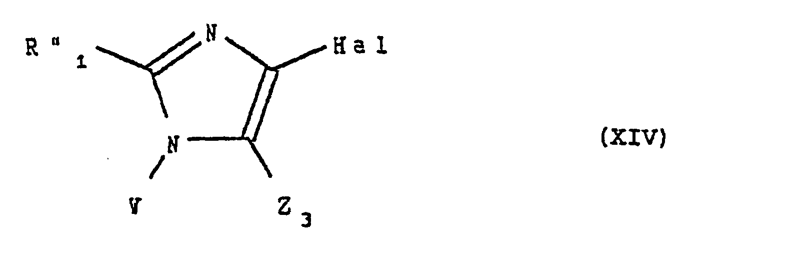 zu erhalten, in denen R'₁, R"₁, Hal, Y' und W die oben angegebenen Bedeutungen besitzen und Z₃ einen Rest Carboxy, Formyl, S-Z₁ wie oben definiert oder den Rest K-O-alk bedeutet, worin K den Rest 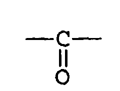 oder 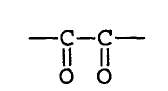 darstellt und alk die oben angegebene Bedeutung besitzt,
und man die Verbindungen der Formel (I₁) oder (XIV), wenn Z₃ einen Rest Formyl oder einen veresterten Rest Carboxy darstellt, einer Reaktion mit einer Verbindung der Formel (VII)
Z₂-S-M (VII)
unterziehen kann, in der S ein Schwefelatom darstellt, M ein Metall bedeutet wie Natrium, Kalium oder Kupfer und Z₂ einen Rest Alkyl, Alkenyl oder Aryl darstellt, gegebenenfalls substituiert, und worin die eventuellen reaktiven Funktionen gegebenenfalls durch Schutzgruppen geschützt sind, um jeweils die Verbindung der Formel (I₂) 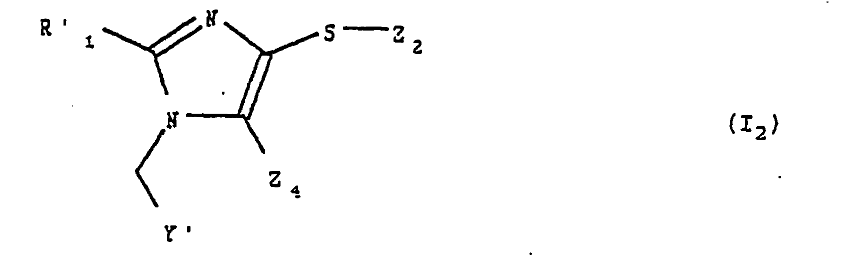 oder die Verbindung der Formel (XV) 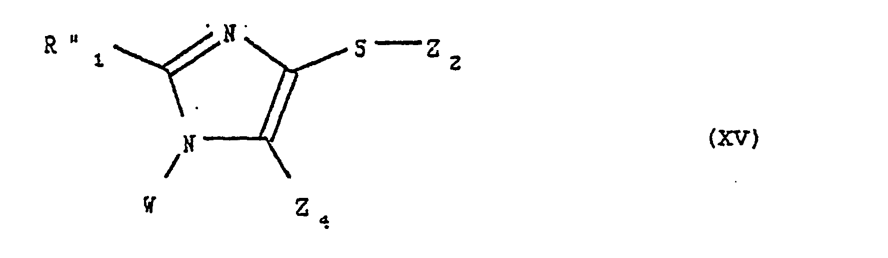 zu erhalten, in denen R'₁, R"₁, Y', Z₂ und W die oben angegebenen Bedeutungen besitzen und Z₄ den Rest Formyl oder verestertes Carboxy darstellt,
und man die Produkte der Formeln (I₂) und (XV), wenn Z₄ den Rest Formyl bedeutet, einer Reaktion zur Oxidation oder Reduktion unterziehen kann, um jeweils ein Produkt der Formel (I₃) 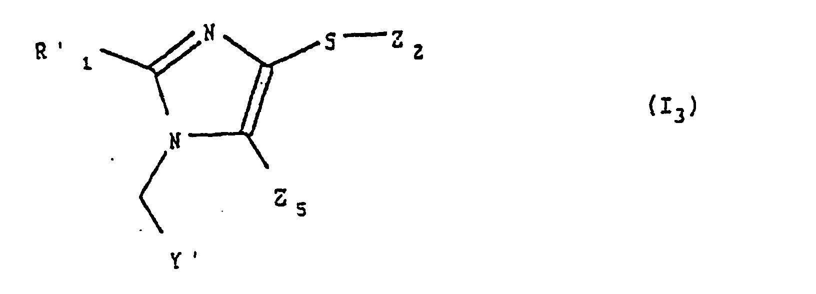 oder ein Produkt der Formel (I₄) 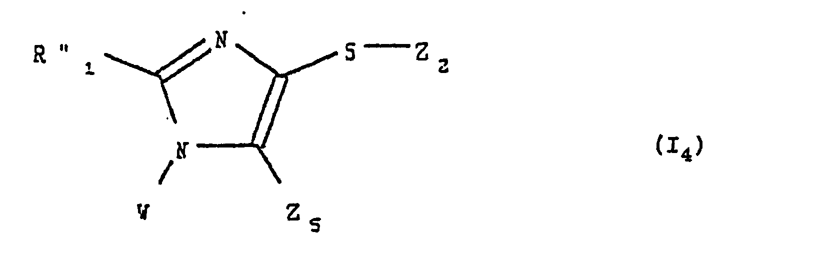 zu erhalten, in denen R'₁, R"₁, Z₂, Y' und W die oben angegebenen Bedeutungen besitzen und Z₅ den Rest CH₂OH oder den Rest Carboxy, frei oder verestert durch einen linearen oder verzweigten Rest Alkyl mit höchstens 6 Kohlenstoffatomen, darstellt,
und man die Produkte der Formeln (XV) oder (I₄) in dem Fall, wo W den Rest P darstellt, wie oben definiert, und nach Freisetzung der durch P, wie oben definiert, blockierten Aminfunktion mit der Verbindung der Formel (III), wie oben definiert, zur Reaktion bringt, um ein Produkt der Formel (I₅) 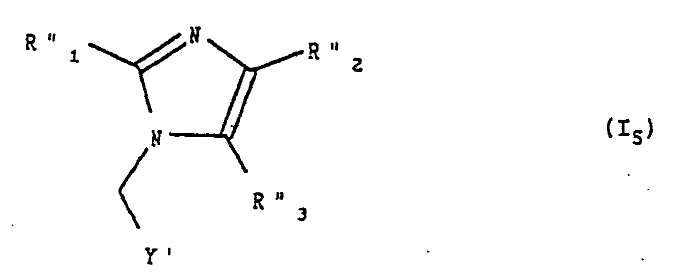 zu erhalten, in der R"₁ und Y' die oben angegebenen Bedeutungen besitzen und eines von R"₂ und R"₃, unabhängig voneinander, Z₄ oder Z₅, wie oben definiert, darstellt und das andere S-Z₂, wie oben definiert, bedeutet,
oder eine Verbindung der Formel (XVI) 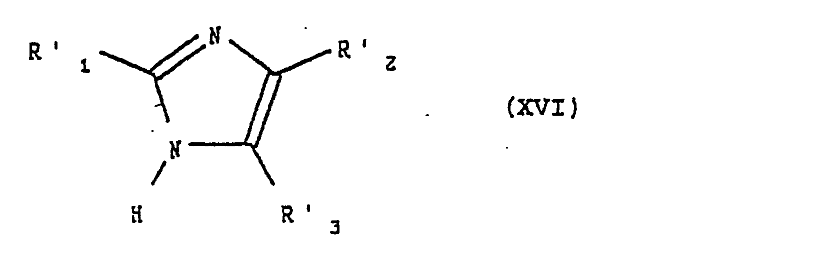 in der R₁ die oben angegebene Bedeutung besitzt und R'₂ und R'₃ die in Anspruch 1 jeweils für R₂ und R₃ angegebenen Bedeutungen besitzen und worin die eventuellen reaktiven Funktionen gegebenenfalls durch Schutzgruppen geschützt sind, einer Reaktion mit der Verbindung der Formel (III), wie oben definiert, unterzieht, um ein Produkt der Formel (I') 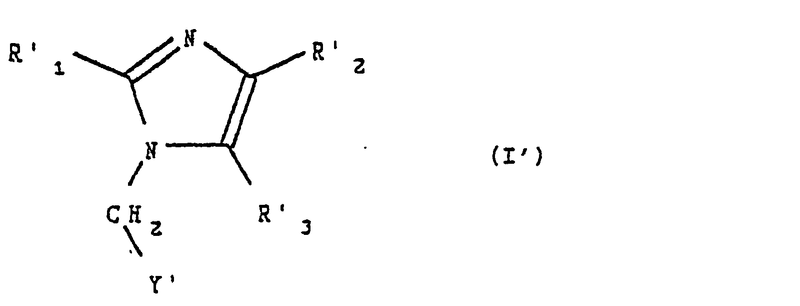 zu erhalten, in der R'₁, R'₂, R'₃ und Y' die oben angegebenen Bedeutungen besitzen, und man die Produkte der Formeln (I₁), (I₂), (I₃), (I₄), (XIV), (XV), (I₅) und (I'), die Produkte der Formel (I) sein können, um diese oder andere Produkte der Formel (I) zu erhalten, wenn erwünscht und wenn erforderlich, einer oder mehreren der folgenden Reaktionen zur Umwandlung in irgendeiner Reihenfolge unterziehen kann:
a) einer Reaktion zur Veresterung der Säurefunktion,
b) einer Reaktion zur Verseifung der Esterfunktion zur Säurefunktion,
c) einer Reaktion zur Umwandlung der Esterfunktion zur Acylfunktion,
d) einer Reaktion zur Umwandlung der Cyanofunktion zur Säurefunktion,
e) einer Reaktion zur Umwandlung der Säurefunktion zur Amidfunktion und anschließend gegebenenfalls zur Thioamidfunktion,
f) einer Reaktion zur Reduktion der Carboxyfunktion zur Alkoholfunktion,
g) einer Reaktion zur Umwandlung der Alkoxyfunktion zur Hydroxylfunktion, oder auch der Hydroxylfunktion zur Alkoxyfunktion,
h) einer Reaktion zur Oxidation der Alkoholfunktion zur Aldehydfunktion, Säurefunktion oder Ketonfunktion,
i) einer Reaktion zur Umwandlung des Restes Formyl in den Rest Carbamoyl,
j) einer Reaktion zur Umwandlung des Restes Carbamoyl in den Rest Nitril,
k) einer Reaktion zur Umwandlung des Restes Nitril in den Rest Tetrazolyl,
l) einer Reaktion zur Oxidation der Gruppe Alkylthio oder Arylthio zum entsprechenden Sulfoxid oder Sulfon,
m) einer Reaktion zur Umwandlung der Sulfidfunktion, Sulfoxidfunktion oder Sulfonfunktion zur entsprechenden Sulfoximinfunktion,
n) einer Reaktion zur Umwandlung der Oxofunktion zur Thioxofunktion,
o) einer Reaktion zur Umwandlung des Restes 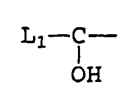 in den Rest 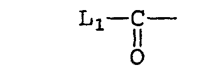 und anschließend, wenn erforderlich, von neuem in den Rest 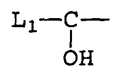 mit L₁ wie oben definiert,
p) einer Reaktion zur Umwandlung der Säurefunktion in die Funktion 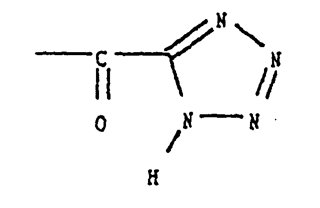
q) einer Reaktion zur Umwandlung der Funktion β-Keto-sulfoxid in die Funktion α-Keto-thioester
r) einer Reaktion zur Umwandlung eines Carbamates zu Harnstoff und insbesondere eines Sulfonylcarbamates zu Sulfonylharnstoff,
s) einer Reaktion zur Entfernung der Schutzgruppen, die von den geschützten reaktiven Funktionen getragen werden können,
t) einer Reaktion zur Salzbildung durch eine Mineralsäure oder organische Säure oder durch eine Base, um das entsprechende Salz zu erhalten,
u) einer Reaktion zur Aufspaltung der racemischen Formen in die gespaltenen Produkte,
wobei die auf diese Weise erhaltenen Produkte der Formel (I) in allen möglichen isomeren racemischen Formen, Enantiomeren und Diastereoisomeren vorliegen können.

5. Als Arzneimittel die Produkte wie in der Formel (I) in Anspruch 1 definiert, wobei die genannten Produkte der Formel (I) in allen möglichen isomeren racemischen Formen, Enantiomeren und Diastereoisomeren vorliegen können, sowie die Additionssalze der genannten Produkte der Formel (I) mit pharmazeutisch akzeptablen Mineralsäuren und organischen Säuren oder mit Mineralbasen und organischen Basen.

6. Als Arzneimittel die Produkte wie in der Formel (I) in Anspruch 2 und 3 definiert, sowie die Additionssalze der genannten Produkte der Formel (I) mit pharmazeutisch akzeptablen Mineralsäuren und organischen Säuren oder mit Mineralbasen und organischen Basen.

7. Pharmazeutische Zusammensetzungen, enthaltend als Wirkstoff mindestens eines der Arzneimittel wie in irgendeinem der Ansprüche 5 oder 6 definiert.

8. Verwendung der Produkte der Formel (I) wie in Anspruch 1 definiert zur Herstellung von pharmazeutischen Zusammensetzungen, vorgesehen für die Behandlung von Erkrankungen, die aus einer anormalen Stimulierung der Rezeptoren von Endothelin resultieren.

9. Verwendung der Produkte der Formel (I) wie in Anspruch 1 definiert zur Herstellung von pharmazeutischen Zusammensetzungen, vorgesehen für die Behandlung von durch Endothelin induzierten Bluthochdruck, von allen vasculären Spasmen und die Behandlung von cerebralen Post-Hämorrhagien und Niereninsuffizienzen, von Herzinfarkt und für die Vorbeugung von post-angioplastischen Restenosen.
